(19) Europäisches Patentamt
European Patent Office
Office européen des brevets

(11) Veröffentlichungsnummer: **0 363 320**
**A2**

# (12) EUROPÄISCHE PATENTANMELDUNG

(21) Anmeldenummer: **89810731.3**

(22) Anmeldetag: **26.09.89**

(51) Int. Cl.[5]: **C07D 473/00 , A61K 31/52**

Claims for the following Contracting States: ES + GR.

(30) Priorität: **06.10.88 CH 3731/88**

(43) Veröffentlichungstag der Anmeldung:
**11.04.90 Patentblatt 90/15**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Allgeier, Hans, Dr.**
**Lichsenweg 20**
**D-7850 Lörrach-Haagen(DE)**

## (54) Substituierte 9H-Purine.

(57) Die Erfindung betrifft neue 9H-Purinderivate, insbesondere die neuen substituierten 9-Benzyl-9H-Purine der allgemeinen Formel

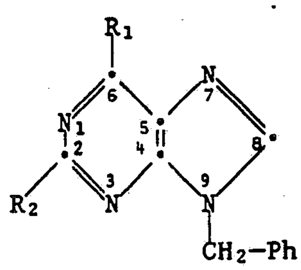

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, mit der Massgabe, dass $R_2$ von Halogen verschieden ist, wenn Ph 2-Fluorphenyl oder 2,5- oder 2,6-Difluorphenyl ist und $R_1$ einen Rest der Formel $-N(R_{11})(R_{12})$ (Ia), worin entweder $R_{11}$ Wasserstoff, Methyl oder Aethyl ist und $R_{12}$ Wasserstoff, Methyl, Hydroxymethyl, Aethyl, n-Propyl, Isopropyl, Cyclopropyl, Cyclopentyl oder Cyclopropylmethyl ist oder worin $R_{11}$ Wasserstoff ist und $R_{12}$ Methoxymethyl ist, darstellt, und mit der weiteren Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, sowie die neuen Verbindungen der Formel I, worin Ph 2-Fluorphenyl oder 2,6-Difluorphenyl ist, $R_1$ N-Methylamino oder N,N-Dimethylamino ist und $R_2$ für Chlor steht, und jeweils ihre Salze. Diese Verbindungen und ihre Salze können als pharmazeutische Wirkstoffe verwendet werden und sind in an sich bekannter Weise herstellbar.

EP 0 363 320 A2

## Substituierte 9H-Purine

Die Erfindung betrifft neue 9H-Purinderivate, insbesondere die neuen substituierten 9-Benzyl-9H-Purine der allgemeinen Formel

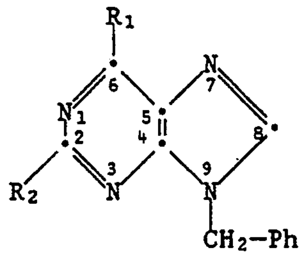

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, mit der Massgabe, dass $R_2$ von Halogen verschieden ist, wenn Ph 2-Fluorphenyl oder 2,5- oder 2,6-Difluorphenyl ist und $R_1$ einen Rest der Formel $-N(R_{11})(R_{12})$ (Ia), worin entweder $R_{11}$ Wasserstoff, Methyl oder Aethyl ist und $R_{12}$ Wasserstoff, Methyl, Hydroxymethyl, Aethyl, n-Propyl, Isopropyl, Cyclopropyl, Cyclopentyl oder Cyclopropylmethyl ist oder worin $R_{11}$ Wasserstoff ist und $R_{12}$ Methoxymethyl ist, darstellt, und mit der weiteren Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, sowie die neuen Verbindungen der Formel I, worin Ph 2-Fluorphenyl oder 2,6-Difluorphenyl ist, $R_1$ H-Methylamino oder N,N-Dimethylamino ist und $R_2$ für Chlor steht, und jeweils ihre Salze, ein Verfahren zur Herstellung solcher Verbindungen oder ihrer Salze, solche Verbindungen oder ihre Salze enthaltende pharmazeutische Präparate und die Verwendung solcher Verbindungen und ihrer Salze.

Der Phenylrest Ph kann bis und mit 3, vorzugsweise 1 oder 2, Halogensubstituenten aufweisen, wobei im Falle von mehrfacher Substitution die Substituenten gleich oder verschieden sein können. Die Substituenten sind jeweils vorzugsweise in einer ortho-Stellung oder in zweiter Linie in einer meta-Stellung gebunden, können jedoch auch in para-Stellung gebunden sein. Als Beispiele seien genannt: 2-Halogenphenyl, ferner 3-und 4-Halogenphenyl, und 2,6-Dihalogenphenyl, ferner 2,3- und 2,5-Di-sowie 2,3,6- und 2,5,6-Trihalogenphenyl. Besonders bevorzugt sind 2-Halogenphenyl und 2,6-Dihalogenphenyl.

Aliphatisch, cycloaliphatisch, cycloaliphatisch-aliphatisch und/oder durch Acyl substituierte Aminogruppen $R_1$ bzw. $R_2$ sind beispielsweise durch einen aliphatischen, cycloaliphatischen oder cycloaliphatisch-aliphatischen Rest oder durch Acyl monosubstituierte oder durch aliphatische, cycloaliphatische, cycloaliphatisch-aliphatische Reste oder durch einen aliphatischen Rest sowie einen cycloaliphatischen Rest oder Acyl disubstituierte Aminogruppen. Dabei kommen als aliphatische Reste beispielsweise Niederalkyl, Niederalkoxyniederalkyl und Hydroxyniederalkyl, als cycloaliphatische Reste z.B. Cycloalkyl, als cycloaliphatisch-aliphatische Reste z.B. Cycloalkylniederalkyl und als Acyl beispielsweise Niederalkanoyl in Betracht. Als Reste $R_1$ bzw. $R_2$ seien beispielsweise genannt: Amino, N-Mono- sowie N,N-Diniederalkylamino, N-(Niederalkoxyniederalkyl)amino, N-(Hydroxyniederalkyl)amino, N-(Hydroxyniederalkyl)-N-niederalkyl-amino, N-Mono- sowie N,N-Dicycloalkylamino, N-Cycloalkyl-N-niederalkyl-amino, N-Mono- sowie N,N-Di-(cycloalkylniederalkyl)amino, N-(Cycloalkylniederalkyl)-N-niederalkyl-amino, N-Niederalkanoylamino und N-Niederalkanoyl-N-niederalkyl-amino.

Vor- und nachstehend sind unter mit "Nieder" bezeichneten organischen Gruppen und Verbindungen, sofern nicht abweichend definiert, vorzugsweise solche zu verstehen, die bis und mit 7, insbesondere bis und mit 4, Kohlenstoffatome (C-Atome) enthalten.

Halogen ist beispielsweise Halogen mit einer Atomnummer bis und mit 35, wie Fluor oder Chlor oder in zweiter Linie Brom.

Niederalkyl ist beispielsweise $C_1$-$C_4$-Alkyl, wie Methyl, Aethyl, n-Propyl, Isopropyl oder n-Butyl, ferner Sekundärbutyl, Isobutyl oder Tertiärbutyl, kann aber auch eine $C_5$-$C_7$-Alkylgruppe, d.h. eine Pentyl-, Hexyl- oder Heptylgruppe, sein.

Niederalkoxy ist z.B. $C_1$-$C_4$-Alkoxy, wie Methoxy, Aethoxy, n-Propyloxy, Isopropyloxy oder n-Butyloxy,

ferner Sekundärbutyloxy, Isobutyloxy oder Tertiärbutyloxy, kann aber auch eine $C_5$-$C_7$-Alkoxygruppe, d.h. eine Pentyl-, Hexyl- oder Heptyloxygruppe, sein.

N-Mononiederalkylamino ist beispielsweise N-$C_1$-$C_7$-Alkyl-, insbesondere N-$C_1$-$C_4$-Alkylamino, wie N-Methyl- oder N-Aethylamino.

N,N-Diniederalkylamino ist beispielsweise N,N-Di-$C_1$-$C_7$-alkyl-, insbesondere N,N-Di-$C_1$-$C_4$-alkylamino, wobei jeweils die beiden N-Alkylgruppen gleich oder verschieden sein können, wie N,N-Dimethyl-, N,N-Diäthyl-, N,N-Diisopropyl- oder N-Butyl-N-methylamino.

N-(Niederalkoxyniederalkyl)amino ist beispielsweise N-($C_1$-$C_4$-Alkoxy-, wie Methoxy- oder Aethoxy-, $C_1$-$C_7$-alkyl)-, insbesondere N-($C_1$-$C_4$-Alkoxy-, wie Methoxy- oder Aethoxy-, $C_1$-$C_4$-alkyl)amino, wie N-(Methoxymethyl)amino oder N-(1-Methoxyäthyl)amino.

N-(Hydroxyniederalkyl)amino ist beispielsweise N-(Hydroxy-$C_1$-$C_7$-alkyl)-, insbesondere N-(Hydroxy-$C_1$-$C_4$-alkyl)amino, wie N-(Hydroxymethyl)amino oder N-(1-Hydroxyäthyl)amino.

N-(Hydroxyniederalkyl)-N-niederalkyl-amino ist beispielsweise N-(Hydroxy-$C_1$-$C_7$-alkyl)-N-$C_1$-$C_7$-alkyl-amino, insbesondere N-(Hydroxy-$C_1$-$C_4$-alkyl)-N-$C_1$-$C_4$-alkyl-amino, wie N-(1-Hydroxyäthyl)-N-methyl-amino oder N-(Hydroxymethyl)-N-äthyl-amino.

N-Monocycloalkylamino ist beispielsweise N-$C_3$-$C_8$-Cycloalkylamino, insbesondere N-$C_3$-$C_6$-Cycloalkylamino, wie N-Cyclopropyl- oder N-Cyclohexylamino.

N,N-Dicycloalkylamino ist beispielsweise N,N-Di-$C_3$-$C_8$-cycloalkylamino, insbesondere N,N-Di-$C_3$-$C_6$-Cycloalkylamino, wobei jeweils die beiden N-Cycloalkylgruppen gleich oder verschieden sein können, wie N,N-Dicyclohexyl- oder N-Cyclohexyl-N-cyclopropylamino.

N-Cycloalkyl-N-niederalkyl-amino ist beispielsweise N-$C_3$-$C_8$-Cycloalkyl-N-$C_1$-$C_7$-alkyl-amino, insbesondere N-$C_3$-$C_6$-Cycloalkyl-N-$C_1$-$C_4$-alkylamino, wie N-Cyclopropyl-N-methyl- oder N-Cyclohexyl-N-äthyl-amino.

N-Mono(cycloalkylniederalkyl)amino ist beispielsweise N-($C_3$-$C_8$-Cycloalkyl-$C_1$-$C_7$-alkyl)amino, insbesondere N-($C_3$-$C_6$-Cycloalkyl-$C_1$-$C_4$-alkyl)amino, wie N-(Cyclopropylmethyl)amino oder N-(1-Cyclohexyläthyl)amino.

N,N-Di(cycloalkylniederalkyl)amino ist beispielsweise N,N-Di($C_3$-$C_8$-cycloalkyl-$C_1$-$C_7$-alkyl)amino, insbesondere N,N-Di($C_3$-$C_6$-cycloalkyl-$C_1$-$C_4$-alkyl)amino, wobei jeweils die beiden N-(Cycloalkylalkyl)gruppen gleich oder verschieden sein können, wie N,N-Di(cyclopropylmethyl)amino oder N-(Cyclopropylmethyl)-N-(1-cyclohexyläthyl)-amino.

N-(Cycloalkylniederalkyl)-N-niederalkyl-amino ist beispielsweise N-($C_3$-$C_8$-Cycloalkyl-$C_1$-$C_7$-alkyl)-N-$C_1$-$C_7$-alkyl-amino, insbesondere N-($C_3$-$C_6$-Cycloalkyl-$C_1$-$C_4$-alkyl)-N-$C_1$-$C_4$-alkyl-amino, wie N-(Cyclopropylmethyl)-N-äthyl-amino oder N-(1-Cyclohexyläthyl)-N-methyl-amino.

Niederalkanoyl ist beispielsweise $C_2$-$C_5$-Alkanoyl, wie Acetyl, Propionyl, Butyryl, Isobutyryl oder Pivaloyl.

N-Niederalkanoylamino ist beispielsweise N-$C_2$-$C_5$-Alkanoylamino, wie N-Acetyl-, N-Propionyl-, N-Butyryl- oder N-Pivaloylamino.

N-Niederalkanoyl-N-niederalkyl-amino ist beispielsweise N-$C_2$-$C_5$-Alkanoyl-N-$C_1$-$C_7$-alkyl-amino, insbesondere N-$C_2$-$C_5$-Alkanoyl-N-$C_1$-$C_4$-alkyl-amino, wie N-Acetyl-N-propyl-amino oder N-Butyryl-N-methyl-amino.

Die Verbindungen I können über ihre basischen Zentren Salze bilden. Salze von Verbindungen I sind daher insbesondere entsprechende Säureadditionssalze, vorzugsweise pharmazeutisch verwendbare Säureadditionssalze. Diese werden beispielsweise mit starken anorganischen Protonensäuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet.

Umfasst sind ferner für pharmazeutische Verwendungen ungeeignete Salze, da diese beispielsweise für die Isolierung bzw. Reinigung freier Verbindungen I sowie deren pharmazeutisch verwendbarer Salze verwendet werden können.

Die neuen Verbindungen I und ihre pharmazeutisch verwendbaren Salze besitzen wertvolle pharmakologische Eigenschaften, insbesondere eine ausgeprägte antikonvulsive Wirksamkeit, die sich beispielsweise an der Maus anhand eines deutlichen Metrazol-Antagonismus im Dosisbereich ab etwa 10 mg/kg p.o. sowie an Maus und Ratte anhand einer ausgeprägten Schutzwirkung gegen durch Elektroschock ausgelöste Konvulsionen im Dosisbereich ab etwa 8 mg/kg p.o. zeigen lässt.

Die Verbindungen I und ihre pharmazeutisch verwendbaren Salze sind dementsprechend vorzüglich geeignet zur Behandlung von Konvulsionen verschiedenartiger Genese, beispielsweise zur Behandlung der

Epilepsie. Sie können dementsprechend als antikonvulsive, beispielsweise antiepileptische, Arzneimittelwirkstoffe verwendet werden. Dabei kann auch die gewerbsmässige Herrichtung der Wirksubstanzen eingeschlossen sein.

Die Erfindung betrifft in erster Linie Verbindungen der Formel I, worin, unter Berücksichtigung der vorstehend erwähnten Massgaben, Ph einen durch Halogen substituierten Phenylrest bedeutet, $R_1$ Wasserstoff, Amino, N-Mono- oder N,N-Diniederalkylamino, N-(Niederalkoxyniederalkyl)amino, N-(Hydroxyniederalkyl)amino, N-(Hydroxyniederalkyl)-N-niederalkyl-amino, N-Mono- oder N,N-Dicycloalkylamino, N-Cycloalkyl-N-niederalkyl-amino, N-Mono- oder N,N-Di(cycloalkylniederalkyl)amino, N-(Cycloalkylniederalkyl)-N-niederalkyl-amino, N-Niederalkanoylamino oder N-Niederalkanoyl-N-niederalkyl-amino darstellt und $R_2$ für Halogen, Niederalkoxy, Niederalkyl, Amino, N-Mono- oder N,N-Diniederalkylamino, N-(Niederalkoxyniederalkyl)amino, N-(Hydroxyniederalkyl)amino, N-(Hydroxyniederalkyl)-N-niederalkyl-amino, N-Mono- oder N,N-Dicycloalkylamino, N-Cycloalkyl-N-niederalkyl-amino, N-Mono- oder N,N-Di-(cycloalkylniederalkyl)amino, N-(Cycloalkylniederalkyl)-N-niederalkyl-amino, N-Niederalkanoylamino oder N-Niederalkanoyl-N-niederalkyl-amino steht, sowie die neuen Verbindungen der Formel I, worin Ph 2-Fluorphenyl oder 2,6-Difluorphenyl ist, $R_1$ N-Methylamino oder N,N-Dimethylamino ist und $R_2$ für Chlor steht, und jeweils ihre Salze.

Die Erfindung betrifft vor allem Verbindungen der Formel I, worin, unter Berücksichtigung der vorstehend erwähnten Massgaben, Ph 2-, 3- oder 4-Halogenphenyl, wie 2-Fluor-, 3-Fluor-, 4-Fluor- oder 2-Chlorphenyl, 2,3-, 2,5- oder 2,6-Dihalogenphenyl, wie 2,3-, 2,5- oder 2,6-Difluorphenyl oder 6-Chlor-2-fluorphenyl, oder 2,3,6- oder 2,5,6-Trihalogenphenyl, wie 2,3,6-Trifluorphenyl oder 5-Chlor-2,6-difluor-phenyl, bedeutet, wobei Halogen jeweils Halogen mit einer Atomnummer bis und mit 35 sein kann, $R_1$ Wasserstoff, Amino, N-$C_1$-$C_4$-Alkylamino, wie N-Methyl-oder N-Aethylamino, N,N-Di-$C_1$-$C_4$-alkylamino, wie N,N-Dimethyl-, N,N-Diäthyl- oder N-Butyl-N-methyl-amino, N-$C_3$-$C_6$-Cycloalkylamino, wie N-Cyclopropyl- oder N-Cyclohexylamino, oder N-Niederalkanoylamino, wie N-Acetylamino, darstellt und $R_2$ für Halogen mit einer Atomnummer bis und mit 35, wie Chlor oder Brom, $C_1$-$C_4$-Alkoxy, wie Methoxy oder Aethoxy, $C_1$-$C_4$-Alkyl, wie Methyl oder Aethyl, Amino, N-$C_1$-$C_4$-Alkylamino, wie N-Methyl- oder N-Aethylamino, N,N-Di-$C_1$-$C_4$-alkylamino, wie N,N-Dimethyl-, N,N-Diäthyl-oder N-Butyl-N-methyl-amino, N-$C_3$-$C_6$-Cycloalkylamino, wie N-Cyclopropyl-oder N-Cyclohexylamino, oder N-Niederalkanoylamino, wie N-Acetylamino, steht, sowie die neuen Verbindungen der Formel I, worin Ph 2-Fluorphenyl oder 2,6-Difluorphenyl ist, $R_1$ N-Methylamino oder N,N-Dimethylamino ist und $R_2$ für Chlor steht, und jeweils ihre Salze.

Die Erfindung betrifft in besonderer Weise Verbindungen der Formel I, worin, unter Berücksichtigung der vorstehend erwähnten Massgaben, Ph 2-Halogenphenyl, wie 2-Chlor- oder 2-Fluorphenyl, oder 2,6-Dihalogenphenyl, wie 2,6-Difluorphenyl, bedeutet, wobei Halogen jeweils Halogen mit einer Atomnummer bis und mit 35 sein kann, $R_1$ Wasserstoff, Amino, N-$C_1$-$C_4$-Alkylamino, wie N-Methyl- oder N-Aethylamino, N,N-Di-$C_1$-$C_4$-alkylamino, wie N,N-Dimethyl-, N,N-Diäthyl- oder N-Butyl-N-methylamino, oder N-Niederalkanoylamino, wie N-Acetylamino, darstellt und $R_2$ für Halogen mit einer Atomnummer bis und mit 35, wie Chlor oder Brom, Amino, N-$C_1$-$C_4$-Alkylamino, wie N-Methyl- oder N-Aethylamino, oder N,N-Di-$C_1$-$C_4$-alkylamino, wie N,N-Dimethyl-, N,N-Diäthyl- oder N-Butyl-N-methyl-amino, steht, sowie die neuen Verbindungen der Formel I, worin Ph 2-Fluorphenyl oder 2,6-Difluorphenyl ist, $R_1$ N-Methylamino oder N,N-Dimethylamino ist und $R_2$ für Chlor steht, und jeweis ihre Salze.

Die Erfindung betrifft in ganz besonderer Weise Verbindungen der Formel 1, worin, unter Berücksichtigung der vorstehend erwähnten Massgaben, Ph 2-Fluorphenyl, 2-Chlorphenyl oder 2,6-Difluorphenyl bedeutet, $R_1$ Wasserstoff, Amino, N-$C_1$-$C_4$-Alkylamino, wie N-Methylamino, oder N,N-Di-$C_1$-$C_4$-alkylamino, wie N,N-Dimethylamino, darstellt und $R_2$ für Halogen mit einer Atomnummer bis und mit 35, wie Chlor, Amino, N-$C_1$-$C_4$-Alkylamino, wie N-Methyl- oder N-Aethylamino, oder N,N-Di-$C_1$-$C_4$-alkylamino, wie N,N-Dimethylamino, steht, sowie die neuen Verbindungen der Formel I, worin Ph 2-Fluorphenyl oder 2,6-Difluorphenyl ist, $R_1$ N-Methylamino oder N,N-Dimethylamino ist und $R_2$ für Chlor steht, und jeweils ihre Salze.

Die Erfindung betrifft vor allem Verbindungen der Formel I, worin, unter Berücksichtigung der vorstehend erwähnten Massgaben, Ph 2-Fluorphenyl oder 2,6-Difluorphenyl bedeutet, $R_1$ N-$C_1$-$C_4$-Alkylamino, wie N-Methylamino, oder N,N-Di-$C_1$-$C_4$-alkylamino, wie N,N-Dimethylamino, darstellt und $R_2$ für Halogen mit einer Atomnummer bis und mit 35, wie Chlor, Amino, N-$C_1$-$C_4$-Alkylamino, wie N-Methyl- oder N-Aethylamino, oder N,N-Di-$C_1$-$C_4$-alkylamino, wie N,N-Dimethylamino, steht, sowie die neuen Verbindungen der Formel I, worin Ph 2-Fluorphenyl oder 2,6-Difluorphenyl ist, $R_1$ N-Methylamino oder N,N-Dimethylamino ist und $R_2$ für Chlor steht, und jeweils ihre Salze.

Die Erfindung betrifft insbesondere Verbindungen der Formel I, worin, unter Berücksichtigung der vorstehend erwähnten Massgaben, Ph 2-Fluorphenyl bedeutet, $R_1$ N,N-Di-$C_1$-$C_4$-alkylamino, wie N,N-Dimethylamino, darstellt und $R_2$ für Halogen mit einer Atomnummer bis und mit 35, wie Chlor, Amino oder

N-$C_1$-$C_4$-Alkylamino, wie N-Methyl- oder N-Aethylamino, steht, sowie die neue Verbindung der Formel I, worin Ph 2-Fluorphenyl ist, $R_1$ N,N-Dimethylamino ist und $R_2$ für Chlor steht, und jeweils ihre Salze.

Die Erfindung betrifft in allererster Linie Verbindungen der Formel I, worin Ph 2-Fluorphenyl bedeutet, $R_1$ N,N-Di-$C_1$-$C_4$-alkylamino, wie N,N-Dimethylamino, darstellt und $R_2$ für N-$C_1$-$C_4$-Alkylamino, wie N-Methylamino, steht, und ihre Salze.

Die Erfindung betrifft namentlich die in den Beispielen genannten neuen Verbindungen der Formel I und ihre Salze.

Ebenfalls Gegenstand der vorliegenden Erfindung ist ein Verfahren zur Herstellung einer Verbindung I oder eines Salzes davon, das auf an sich bekannten Verfahrensweisen beruht und z.B. dadurch gekennzeichnet ist, dass man

a) in einer Verbindung der Formel

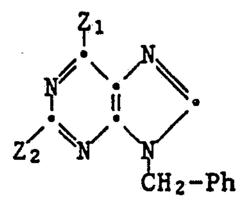

(II),

worin $Z_1$ eine nucleofuge Abgangsgruppe $X_1$ ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abgangsgruppe $X_2$ steht, oder einem Tautomeren und/oder Salz davon $Z_1$ in $R_1$ und/oder $Z_2$ in $R_2$ überführt oder

b) aus einer Verbindung der Formel

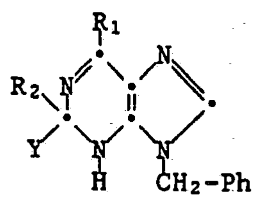

(III),

worin Y Hydroxy, Mercapto oder gegebenenfalls aliphatisch substituiertes Amino bedeutet, oder einem Tautomeren und/oder Salz davon die Verbindung Y-H eliminiert oder

c) zur Herstellung einer Verbindung I, worin $R_2$ für Amino steht, oder eines Salzes davon eine Verbindung der Formel

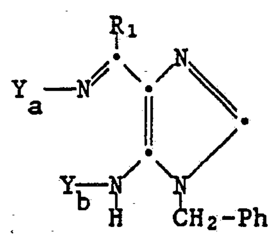

(IV),

worin einer der Reste $Y_a$ und $Y_b$ Cyano und der andere Wasserstoff bedeutet, oder ein Tautomeres und/oder Salz davon cyclisiert oder

d) eine Verbindung der Formel

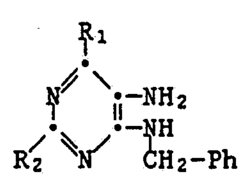

(V)

oder ein Salz davon mit Ameisensäure oder einem reaktionsfähigen Derivat davon umsetzt oder

e) eine Verbindung der Formel

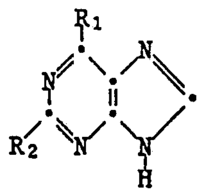

(VI)

oder ein Salz davon mit einer Verbindung der Formel $X_1$-$CH_2$-Ph (VII), worin $X_1$ eine nucleofuge Abgangsgruppe darstellt, umsetzt oder

f) zur Herstellung einer Verbindung I, worin mindestens einer der Reste $R_1$ und $R_2$ für Amino steht, oder eines Salzes davon in einer Verbindung der Formel

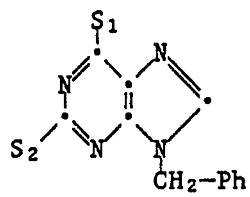

(VIII),

worin $S_1$ eine Aminoschutzgruppe bedeutet und $S_2$ eine Aminoschutzgruppe oder ein Rest $R_2$ ist oder worin $S_1$ einen Rest $R_1$ darstellt und $S_2$ eine Aminoschutzgruppe bedeutet, oder einem Salz davon $S_1$ in $R_1$ und/oder $S_2$ in $R_2$ überführt und gewünschtenfalls jeweils ein gegebenenfalls verfahrensgemäss erhältliches Isomerengemisch in die Komponenten auftrennt und das Isomere der Formel I isoliert, eine verfahrensgemäss oder auf andere Weise erhältliche Verbindung I in eine andere Verbindung I überführt, eine verfahrensgemäss erhältliche freie Verbindung I in ein Salz überführt oder ein verfahrensgemäss erhältliches Salz einer Verbindung I in die freie Verbindung I oder in ein anderes Salz der Verbindung I umwandelt und/oder ein gegebenenfalls verfahrensgemäss erhältliches Stereoisomerengemisch in die Stereoisomeren auftrennt und das gewünschte Stereoisomere isoliert.

Die vor- und nachstehend beschriebenen verfahrensgemässen Reaktionen sowie die Herstellung neuer Ausgangsstoffe bzw. Zwischenprodukte erfolgen in an sich bekannter Weise. Dabei wird in Analogie zur Reaktions- und Bildungsweise bekannter Ausgangsstoffe bzw. Zwischenprodukte, auch wenn nicht ausdrücklich erwähnt, unter den jeweils üblichen Reaktionsbedingungen, z.B. je nach Bedarf unter Kühlen, bei Raumtemperatur oder unter Erwärmen, beispielsweise in einem Temperaturbereich von etwa -10°C bis etwa +250°C, vorzugsweise von etwa 20°C bis etwa 200°C, unter Verwendung der jeweils üblichen Hilfsmittel, wie Katalysatoren, Kondensations- sowie Solvolysemittel, in Ab- oder üblicherweise in Anwesenheit eines geeig neten Lösungs- bzw. Verdünnungsmittels oder eines Gemisches derselben, gegebenenfalls in einem geschlossenen Gefäss, in einer Inertgasatmosphäre und/oder unter wasserfreien Bedingungen, gearbeitet.

Das vor- und nachstehend aufgeführte Ausgangsmaterial der Formeln II, III, IV, V, VI, VII und VIII, das für die Herstellung der Verbindungen I und ihrer Salze verwendet wird, ist entweder bekannt oder kann ebenfalls nach an sich bekannten Methoden hergestellt werden, ebenso wie alle weiteren im Rahmen der vorliegenden Beschreibung erwähnten Ausgangsmaterialien und Zwischenprodukte.

Ausgangsmaterial mit basischen Zentren, beispielsweise die Verbindungen II, III, IV, V und VIII bzw. gegebenenfalls ihre Tautomeren, kann auch in Form von Salzen, wie Säureadditionssalzen, vorliegen, vorzugsweise mit starken anorganischen oder organischen Säuren, z.B. analog der vorstehend für Säureadditionssalze von Verbindungen I genannten Art. Analoges gilt auch für alle weiteren im Rahmen der vorliegenden Beschreibung erwähnten Ausgangsmaterialien bzw. Zwischenprodukte mit basischen Zentren.

Tautomere von Verbindungen II bzw. deren Salzen können beispielsweise dann auftreten, wenn die Gruppen $Z_1$ und/oder $Z_2$ Hydroxy oder Mercapto darstellen. Demnach können z.B. Verbindungen II bzw. deren Salze mit Enol- und/oder Enthiol-Teilstrukturen auch in protomerer Form, d.h. als entsprechende Oxo- und/oder Thioxotautomere vorliegen und/oder mit diesen in einem dynamischen Gleichgewicht stehen. Analoges gilt auch für alle weiteren im Rahmen der vorliegenden Beschreibung erwähnten Tautomeren von Ausgangsmaterialien bzw. Zwischenprodukten und deren Salzen, wobei zusätzlich gegebenenfalls vorhandene Enamin-Teilstrukturen auch in der tautomeren Iminoform vorliegen können.

Nucleofuge Abgangsgruppen $X_1$ bzw. $X_2$ in gemäss der Verfahrensvariante a) verwendeten Ausgangsverbindungen II sind beispielsweise gegebenenfalls verätherte oder veresterte Hydroxy- oder Mercaptogruppen, Sulfinyl- und Sulfonylgruppen, ferner Sulfoniumgruppen. Veräthertes Hydroxy ist beispielsweise gegebenenfalls substituiertes Phenylniederalkoxy, wie gegebenenfalls substituiertes Benzyloxy. Verestertes Hydroxy bedeutet insbesondere mit einer organischen Sulfonsäure verestertes Hydroxy, wie gegebenenfalls durch Halogen substituiertes Niederalkansulfonyloxy, z.B. Methan- oder Trifluormethansulfonyloxy, Cycloalkansulfonyloxy, z.B. Cyclohexansulfonyloxy, oder gegebenenfalls durch Niederalkyl oder Halogen substituiertes Benzolsulfonyloxy, z.B. Benzol-, p-Bromphenyl- oder p-Toluolsulfonyloxy, ferner Niederalkanoyloxy, z.B. Acetoxy. Veräthertes Mercapto ist z.B. Niederalkylthio, wie Methylthio, gegebenenfalls substituiertes Arylthio, wie gegebenenfalls substituiertes Phenyl- oder Naphthylthio, beispielsweise Phenyl-, p-Tolyl- oder Naphthylthio, oder gegebenenfalls substituiertes Arylniederalkylthio, wie gegebenenfalls substituiertes Benzyl- oder Naphthylmethylthio, z.B. Benzyl-, p-Brombenzyl- oder Naphthylmethylthio. Veresterte Mercaptogruppen sind beispielsweise Niederalkanoylthiogruppen, wie Acetylthio. Sulfinylgruppen sind beispielsweise Niederalkansulfinylgruppen, wie Methansulfinyl, gegebenenfalls substituierte Arylsulfinylgruppen, wie gegebenenfalls substituiertes Benzol- oder Naphthylsulfinyl, beispielsweise p-Toluol-oder Naphthylsulfinyl, oder gegebenenfalls substituiertes Benzylsulfinyl, wie Benzyl- oder p-Chlorbenzylsulfinyl. Sulfonylgruppen sind beispielsweise Niederalkansulfonylgruppen, wie Methansulfonyl, gegebenenfalls substituierte Arylsulfonylgruppen, wie gegebenenfalls substituiertes Benzol- oder Naphthylsulfonyl, beispielsweise Benzol- oder Naphthylsulfonyl, oder gegebenenfalls substituiertes Benzylsulfonyl, wie Benzyl- oder p-Methylbenzylsulfonyl. Sulfoniumgruppen sind z.B. Diniederalkylsulfoniumgruppen, wie Dimethylsulfonium. Nucleofuge Abgangsgruppen $X_1$ sind vorteilhaft auch mit einem Niederalkanol verätherte Hydroxygruppen, wie Methoxy oder Aethoxy, oder mit einer Mineralsäure veresterte Hydroxygruppen, vor allem Halogen, wie Chlor, Brom oder Iod.

Die Ueberführung von $X_1$ und/oder $X_2$ in Verbindungen II, deren Tautomeren und jeweils ihren Salzen in Reste $R_1$ und/oder $R_2$ erfolgt durch Umsetzung mit Verbindungen der Formel H-$R_1$ (IIb) und/oder H-$R_2$ (IIj) oder gegebenenfalls jeweils einem Salz davon unter Abspaltung von Verbindungen $X_1$-H und/oder $X_2$-H, wobei in üblicher Weise, beispielsweise unter Kühlen, bei Raumtemperatur oder unter Erwärmen, z.B. im Temperaturbereich von etwa -20 bis etwa +250° C, vorzugsweise von etwa -10 bis etwa +200° C, gegebenenfalls in Gegenwart eines inerten Lösungs- oder Verdünnungsmittels oder eines Gemisches derselben, gegebenenfalls in Gegenwart eines wasserbindenden Mittels, gegebenenfalls in Gegenwart eines basischen Mittels und/oder unter Inertgas, wie Stickstoff, gearbeitet wird.

Als inerte Lösungs- oder Verdünnungsmittel sind beispielsweise Wasser, cyclische Aether, aromatische Kohlenwasserstoffe, N,N-Diniederalkylniederalkansäureamide, Phosphorsäureniederalkylamide, Diniederalkylsulfoxide, cyclische Amine und insbesondere, gegebenenfalls in Form von Gemischen mit Wasser, Niederalkanole, wie Tetrahydrofuran, Dioxan, Benzol, Toluol, Xylol, N,N-Dimethylformamid, Hexamethylphosphorsäuretriamid, Dimethylsulfoxid, N-Methylmorpholin und insbesondere, gegebenenfalls in Form von Gemischen mit Wasser, Methanol und Aethanol, zu nennen. Ebenso können zur Herstellung von Verbindungen I, worin mindestens einer der Reste $R_1$ und $R_2$ eine gegebenenfalls wie angegeben substituierte Aminogruppe darstellt, bzw. ihren Salzen auch Ammoniak bzw. entsprechende Amine H-$R_1$ (IIb; $R_1$ = wie angegeben substituiertes Amino) und/oder H-$R_2$ (IIj; $R_2$ = wie angegeben substituiertes Amino) im Ueberschuss eingesetzt und als Lösungs- oder Verdünnungsmittel und/oder Cosolvens, gegebenenfalls auch in gelöster Form, z.B. als wässrige Lösung, verwendet werden. Analog können auch entsprechende Niederalkanole H-$R_2$ (IIj; $R_2$ = Niederalkoxy) im Ueberschuss eingesetzt werden, wenn lediglich Reste $X_2$ in Niederalkoxy $R_2$ überführt werden sollen.

Wasserbindende Mittel sind z.B. Oxide des Phosphors, wie Phosphorpentoxid, Sulfate von Alkali- oder Erdalkalimetallen, wie Natrium- oder Calciumsulfat, Halogenide von Erdalkalimetallen, wie Calciumchlorid, oder Carbodiimide, wie N,N′-Dicyclohexylcarbodiimid.

Basische Mittel sind beispielsweise Alkali- oder Erdalkalimetallhydroxide, -hydride, -amide, -niederalkanolate, -carbonate, -diniederalkylamide oder -niederalkylsilylamide, Niederalkylamine, gegebenenfalls N-niederalkylierte Cycloalkylamine, basische Heterocyclen, Ammoniumhydroxide sowie carbocyclische Amine. Beispielhaft seien Natriumhydroxid, -hydrid, -amid, -methanolat, -äthanolat, Kalium-tert.-butanolat, -carbonat, Lithiumdiisopropylamid, Kalium-bis(trimethylsilyl)-amid, Calciumhydrid, Triäthylamin, Cyclohexylamin, N-Cyclohexyl-N,N-dimethylamin, Pyridin, Benzyl-trimethyl-ammoniumhydroxid sowie 1,5-Diazabicyclo[5.4.0]undec-5-en (DBU) genannt. Vorzugsweise können anstelle eines zusätzlichen basischen Mittels bei der Herstellung von Verbindungen I, worin mindestens einer der Reste $R_1$ und $R_2$ eine gegebenenfalls wie angegeben substituierte Aminogruppe darstellt, bzw. ihren Salzen auch Ammoniak bzw. entsprechende Amine H-$R_1$ (IIb; $R_1$ = wie angegeben substituiertes Amino) und/oder H-$R_2$ (IIj; $R_2$ = wie

angegeben substituiertes Amino) Verwendung finden, die dann vorteilhaft im Ueberschuss eingesetzt werden.

In einer bevorzugten Ausführungsform der Verfahrensvariante a) wird zur Herstellung von Verbindungen I, worin $R_1$ von Wasserstoff verschieden ist, bzw. deren Salzen eine Verbindung der Formel

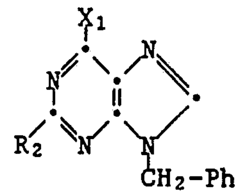

(IIa),

worin $X_1$ eine nucleofuge Abgangsgruppe, vorzugsweise Hydroxy, Halogen, wie Chlor oder Brom, oder, insbesondere zur Herstellung von Verbindungen I oder deren Salzen, worin $R_1$ N,N-Diniederalkylamino ist, eine Sulfonylgruppe, darstellt, oder ein Tautomeres, z.B. eine entsprechende 1H-6-Oxo-Verbindung, und/oder Salz davon mit Ammoniak oder einem Amin der Formel $H-R_1$ (IIb; $R_1$ = wie angegeben substituiertes Amino) oder einem Salz davon unter Abspaltung einer Verbindung $X_1$-H umgesetzt, wobei in üblicher Weise, beispielsweise bei Raumtemperatur oder unter Erwärmen, z.B. im Temperaturbereich von etwa 20 bis etwa 250° C, gegebenenfalls in Gegenwart eines inerten Lösungs- oder Verdünnungsmittels, z.B. der zuvor angegebenen Art, oder eines Gemisches derselben, gegebenenfalls in Gegenwart eines basischen Mittels, beispielsweise der vorstehend erwähnten Art, gegebenenfalls in Gegenwart eines wasserbindenden Mittels, z.B. der zuvor angegebenen Art, und/oder unter Inertgas, wie Stickstoff, gearbeitet wird.

In einer weiteren bevorzugten Ausführungsform der Verfahrensvariante a) wird eine Verbindung der Formel

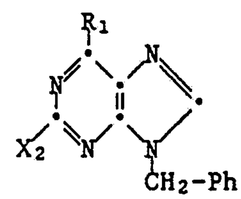

(IIc),

worin $X_2$ eine nucleofuge Abgangsgruppe, vorzugsweise Hydroxy oder, insbesondere zur Herstellung von Verbindungen I oder deren Salzen, worin $R_2$ N,N-Diniederalkylamino ist, eine Sulfonylgruppe, darstellt, oder ein Tautomeres und/oder Salz davon mit einer Verbindung der Formel $H-R_2$ (IIj) oder einem Salz davon unter Abspaltung einer Verbindung $X_2$-H umgesetzt.

Die Ueberführung von $X_2$ in Verbindungen IIc, deren Tautomeren und jeweils ihren Salzen in $R_2$ -(Abspaltung von $X_2$-H) erfolgt in üblicher Weise, zum Beispiel wie vorstehend für die Ueberführung von Verbindungen IIa, deren Tautomeren und jeweils ihren Salzen in Verbindungen I bzw. deren Salze erläutert.

In einer weiteren bevorzugten Ausführungsform der Verfahrensvariante a) wird zur Herstellung von Verbindungen I, worin $R_1$ für Wasserstoff steht, bzw. deren Salzen eine Verbindung IIa, worin $X_1$ eine nucleofuge Abgangsgruppe, vorzugsweise Halogen, wie Chlor oder Brom, oder gegebenenfalls veräthertes Mercapto, wie Niederalkylthio, z.B. Methylthio, darstellt, oder ein Tautomeres und/oder Salz davon reduziert.

Als Reduktionsmittel kommen beispielsweise Raney-Nickel (insbesondere zur Ueberführung von gegebenenfalls veräthertem Mercapto $X_1$ in Wasserstoff $R_1$) oder Wasserstoff in Betracht. Die Reduktion mit Raney-Nickel erfolgt in üblicher Weise, z.B. durch Umsetzung mit einer Lösung des Metalls in einem Niederalkanol, wie Methanol, unter Erwärmen, beispielsweise im Temperaturbereich von etwa 20 bis etwa 140° C, vorzugsweise von etwa 50 bis etwa 100° C. Die Reduktion mit Wasserstoff erfolgt vorteilhaft in Gegenwart eines Hydrierungskatalysators. Als solcher kommt z.B. ein Element der VIII. Nebengruppe des Periodensystems der Elemente oder ein Derivat davon in Betracht, wie Palladium, Platin, Platinoxid, Ruthenium, Rhodium, ein Tris(triphenylphosphan)-rhodium(I)-halogenid, z.B. -chlorid, oder Raney-Nickel, wobei der Katalysator gegebenenfalls auf einem Trägermaterial, wie Aktivkohle, einem Alkalimetallcarbonat oder -sulfat oder einem Kieselgel, aufgezogen sein kann. Die katalytische Hydrierung erfolgt vorzugsweise in einem polaren Lösungs- oder Verdünnungsmittel, insbesondere in einem Niederalkanol, wie Methanol, oder in einer starken anorganischen Säure, wie Halogenwasserstoffsäure, z.B. Chlorwasserstoffsäure, oder

starken organischen Carbonsäure, insbesondere wässriger Essigsäure oder Eisessig, wobei unter Kühlung oder Erwärmen, vorzugweise in einem Temperaturbereich von etwa -10 bis etwa +120 °C, insbesondere bei Raumtemperatur, gearbeitet wird. Die Ueberführung von gegebenenfalls veräthertem Mercapto $X_1$ in Wasserstoff $R_1$ kann auch durch in üblicher Weise vorgenommene Umsetzung einer entsprechenden Verbindung IIa mit einem Triniederalkylphosphit; z.B. Triäthylphosphit, durchgeführt werden.

In einer weiteren bevorzugten Ausführungsform der Verfahrensvariante a) können, ausgehend von einer Verbindung der Formel

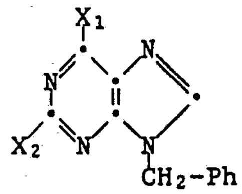

(IId),

worin $X_1$ und $X_2$ jeweils für eine nucleofuge Abgangsgruppe der vorstehend beschriebenen Art, vorzugsweise Hydroxy oder, insbesondere zur Herstellung von Verbindungen I oder deren Salzen, worin $R_1$ und/oder $R_2$ N,N-Diniederalkylamino ist, eine Sulfonylgruppe oder, insbesondere zur Herstellung von Verbindungen I oder deren Salzen, worin $R_1$ Wasserstoff ist, eine gegebenenfalls verätherte Mercaptogruppe oder im Fall von $X_1$ auch Halogen, wie Chlor oder Brom, stehen, oder einem Tautomeren und/oder Salz davon nacheinander die Gruppe $X_1$ durch einen Rest $R_1$ und die Gruppe $X_2$ durch einen Rest $R_2$ ersetzt werden. So kann z.B. vorzugsweise eine Verbindung IId oder ein Tautomeres und/oder Salz davon zunächst mit Ammoniak oder einem Amin der Formel H-$R_1$ (IIb; $R_1$ = wie angegeben substituiertes Amino) oder einem Salz davon umgesetzt oder, beispielsweise mittels Einwirkung von Raney-Nickel oder durch katalytische Hydrierung der zuvor beschriebenen Art, reduziert und das gebildete Zwischen produkt IIc oder ein Tautomeres und/oder Salz davon anschliessend durch Umsetzung mit einer Verbindung H-$R_2$ (IIj) oder einem Salz davon in eine Verbindung der Formel I bzw. ein Salz davon überführt werden.

Die Ueberführung von $X_1$ bzw. $X_2$ in Verbindungen IId, deren Tautomeren und jeweils ihren Salzen in $R_1$ bzw. $R_2$ erfolgt in üblicher Weise, beispielsweise wie vorstehend für die entsprechende Ueberführung von Verbindungen IIa bzw. IIc, deren Tautomeren und jeweils ihren Salzen in Verbindungen I bzw. deren Salze beschrieben, wobei gegebenenfalls das zunächst aus IId entstehende Zwischenprodukt IIc nicht isoliert zu werden braucht, sondern vorteilhaft in situ ohne zusätzliche Reinigung zu einer Verbindung I bzw. deren Salz weiterumgesetzt wird.

Die Verbindungen IIb und IIj bzw. jeweils deren Salze sind bekannt.

Ebenso sind die Verbindungen IId, worin $X_1$ und $X_2$ Hydroxy bedeuten, und deren Tautomere und/oder Salze bekannt oder können in Analogie zu bekannten Methoden hergestellt werden, z.B. entsprechend der Verfahrensvariante e) durch Umsetzung einer Verbindung der Formel

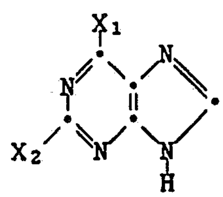

(VI'),

worin $X_1$ und $X_2$ Hydroxy bedeuten, oder eines Tautomeren und/oder Salzes davon mit einer Verbindung VII.

Verbindungen IId, worin $X_1$ und/oder $X_2$ (eine) von Hydroxy verschiedene nucleofuge Abgangsgruppe(n) darstellen, oder deren Tautomere und/oder Salze lassen sich durch in üblicher Weise erfolgende Umsetzung einer Verbindung IId ($X_1 = X_2 =$ Hydroxy) oder eines Tautomeren und/oder Salzes davon mit einer Verbindung der Formel $X_1$-H und/oder $X_2$-H, worin $X_1$ und $X_2$ jeweils eine von Hydroxy verschiedene nucleofuge Abgangsgruppe bedeuten, z.B. im Falle von $X_1$ vorzugsweise Halogen oder gegebenenfalls veräthertes Mercapto, erhalten, wobei Halogen-Abgangsgruppen auch in analoger Weise wie nachfolgend beschrieben durch Umsetzung mit einem Halogenierungsmittel eingeführt werden können.

Die Verbindungen IIa, worin $X_1$ Halogen ist, oder deren Tautomere und/oder Salze lassen sich in Analogie zu bekannten Methoden herstellen, beispielsweise durch Umsetzung einer Verbindung der Formel

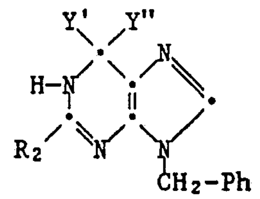

(IIe),

worin Y′ und Y″ gemeinsam für gegebenenfalls funktionell abgewandeltes Oxo $X_3$ stehen, oder eines Tautomeren und/oder Salzes davon mit einem Halogenierungsmittel.

Gegebenenfalls funktionell abgewandeltes Oxo $X_3$ in Verbindungen IIe, deren Tautomeren und jeweils ihren Salzen ist beispielsweise Thioxo oder gegebenenfalls substituiertes Imino, vorzugweise aber Oxo. Gegebenenfalls substituiertes Imino ist beispielsweise eine Gruppe $=N-R_1$, wie Imino, N-Niederalkylimino, N-Cycloalkylimino oder N-Niederalkanoylimino.

Halogenierungsmittel sind beispielsweise Halogenide von Phosphor oder Schwefel, wie Phosphortrihalogenide, Phosphorpentahalogenide, Phosphoroxytrihalogenide, Thionylhalogenide oder Sulfurylhalogenide, z.B. Phosphortrichlorid, -bromid, Phosphorpentachlorid, Phosphoroxytrichlorid, Thionylchlorid oder Sulfurylchlorid, können aber beispielsweise auch Säurehalogenide, wie Säurechloride, der Kohlensäure, beispielsweise Phosgen, sein.

Die Umsetzung einer Verbindung IIe bzw. eines Tautomeren und/oder Salzes davon mit einem Halogenierungsmittel erfolgt unter den üblichen Reaktionsbedingungen, beispielsweise unter Erwärmen, z.B. im Temperaturbereich von etwa 20 bis etwa 200°C, und in einem inerten Lösungsmittel, wie einem Halogenniederalkan, z.B. Tetrachlormethan, vorzugsweise jedoch in Form einer Lösung oder Suspension der Verbindung IIe bzw. eines Tautomeren und/oder Salzes davon in einem Ueberschuss des Halogenierungsmittels.

Verbindungen IIa, worin $X_1$ eine von Halogen verschiedene nucleofuge Abgangsgruppe ist, oder deren Tautomere und/oder Salze lassen sich in üblicher Weise durch Weiterumsetzung einer Verbindung IIa ($X_1$ = Halogen) oder eines Tautomeren und/oder Salzes davon mit einer Verbindung $X_1$-H, worin $X_1$ eine von Halogen verschiedene nucleofuge Abgangsgruppe bedeutet, vorzugsweise gegebenenfalls veräthertes Mercapto, herstellen.

Die Verbindungen IIc oder deren Tautomere und/oder Salze lassen sich in Analogie zu bekannten Methoden aus Verbindungen IId oder deren Tautomeren und/oder Salzen durch Ueberführung von $X_1$ in $R_1$, beispielsweise in der vorstehend beschriebenen Art, herstellen.

Eine Verbindung IIe, worin Y′ und Y″ gemeinsam eine Gruppe $X_3$, vorzugsweise Oxo, darstellen, oder ein Tautomeres und/oder Salz davon ist beispielsweise durch Umsetzung einer Verbindung der Formel

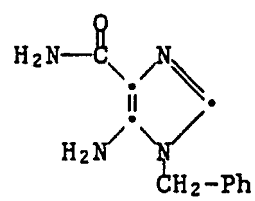

(IIf)

oder eines Salzes davon mit einer Verbindung der Formel $R_2$-X (IIg), worin X für die funktionelle Gruppe einer Carbonsäure oder eines funktionellen Derivates davon, beispielsweise für eine Carboxylgruppe oder für eine Niederalkoxycarbonylgruppe der Formel -C(=O)-O-Alk, worin Alk für Niederalkyl, wie Methyl, steht, insbesondere aber für eine Halogencarbonylgruppe der Formel -C(=O)-Hal, worin Hal für Halogen, wie Chlor oder Brom, steht, eine Amidgruppe der Formel -C(=O)-Am, worin Am für gegebenenfalls substituiertes Amino, beispielsweise Amino, N-Niederalkylamino, wie N-Methylamino, oder N,N-Diniederalkylamino, wie N,N-Dimethyl- oder N,N-Diisopropylamino, steht, oder eine Orthoestergruppe der Formel $-C(O-Alk)_3$, worin Alk Niederalkyl, wie Aethyl, bedeutet, steht, bzw. gegebenenfalls einem Salz davon unter den üblichen Reaktionsbedingungen, beispielsweise in Gegenwart eines Kondensationsmittels, wie eines basischen Mittels, und/oder unter Erwärmen, z.B. im Temperaturbereich von etwa 20 bis etwa 200°C, erhältlich.

Es ist ebenso möglich, in den Verbindungen IIa, IIc und IId z.B. für eine Substitution weniger gut geeignete Abgangsgruppen zunächst in besser geeignete Abgangsgruppen zu überführen, z.B. Niederalkylthio $X_1$ bzw. $X_2$ zu Niederalkansulfonyl zu oxidieren oder Mercapto $X_1$ bzw. $X_2$ zu Niederalkylthio zu veräthern, und anschliessend die Umsetzung mit den Verbindungen IIb bzw. IIj vorzunehmen.

Die Verbindungen IIa, IIc und IId, deren Tautomere und jeweils ihre Salze lassen sich vorteilhaft auch in analoger Weise wie unter der Verfahrensvariante d) beschrieben herstellen, indem man eine entsprechende Verbindung der Formel

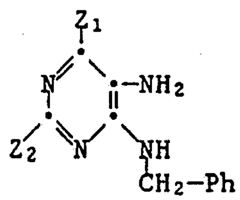

(V'),

worin $Z_1$ eine nucleofuge Abgangsgruppe $X_1$ ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abgangsgruppe $X_2$ steht, oder ein Salz davon in analoger Weise wie unter der Verfahrensvariante d) beschrieben mit Ameisensäure oder einem reaktionsfähigen Derivat davon umsetzt.

Gegebenenfalls aliphatisch substituiertes Amino Y in gemäss der Verfahrensvariante b) verwendeten Ausgangsverbindungen III, deren Tautomeren und jeweils ihren Salzen ist beispielsweise solches, wie es vorstehend bei der Erläuterung der Reste $R_1$ und $R_2$ aufgeführt wird, kann aber auch davon verschiedenes Amino, wie Anilino, sein.

Die Eliminierung der Verbindung Y-H aus Verbindungen III, deren Tautomeren und jeweils ihren Salzen erfolgt in üblicher Weise, beispielsweise in einem inerten Lösungs- oder Verdünnungsmittel, beispielsweise der unter der Verfahrensvariante a) erwähnten Art, durch Erwärmen, z.B. im Temperaturbereich von etwa 40 bis etwa 250°C, vorzugsweise von etwa 80 bis etwa 200°C, und/oder durch Säurebehandlung. Dafür geeignete Säuren sind beispielsweise Mineralsäuren oder deren Anhydride bzw. sauren Salze, z.B. Halogenwasserstoffsäuren, Schwefelsäure, Alkalimetallhydrogen sulfate, Phosphorsäure, Polyphosphorsäure, Phosphorpentoxid, Phosphortrichlorid oder Phosphoroxytrichlorid, organische Sulfonsäuren, wie Methan- oder p-Toluolsulfonsäure, oder Carbonsäuren bzw. deren Anhydride oder Halogenide, wie Niederalkansäuren und deren Anhydride oder Halogenide, z.B. Essigsäure, Acetanhydrid oder Acetylchlorid, ferner gepufferte Säurelösungen, z.B. Phosphat- oder Acetatpuffer, oder Hydrohalogenide von Stickstoffbasen, z.B. Ammonium- oder Pyridiniumchlorid.

In einer bevorzugten Ausführungsform der Verfahrensvariante b) wird beispielsweise eine Verbindung III, worin Y Hydroxy bedeutet, oder ein Tautomeres und/oder Salz davon durch Erwärmen auf 100 bis 200°C in einem inerten Lösungsmittel, beispielsweise einem Niederalkansäureamid, wie Acetamid, unter Austritt eines Aequivalents Wasser in eine Verbindung I bzw. ein Salz davon überführt.

Wegen der leichten Zugänglichkeit der entsprechenden Ausgangsverbindungen III ist die Verfahrensvariante b) insbesondere zur Herstellung von Verbindungen I, worin $R_1$ Amino ist, bzw. deren Salzen geeignet. So lassen sich derartige Ausgangsverbindungen III, deren Tautomere und jeweils ihre Salze in Analogie zu bekannten Methoden erhalten und werden vorzugsweise in situ hergestellt, zum Beispiel durch Cyclisierung einer Verbindung der Formel

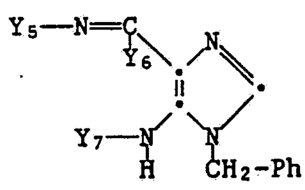

(IIIa),

worin entweder $Y_5$ und $Y_7$ Wasserstoff und $Y_6$ eine Gruppe der Formel $R_2$-C(=$X_3$)-NH- (IIIb) bedeuten oder $Y_5$ und $Y_6$ gemeinsam für eine zusätzliche Bindung und $Y_7$ für eine Gruppe der Formel $R_2$-C(Y)($NH_2$)- (IIIc) stehen, oder eines Tautomeren und/oder Salzes davon, wobei die intermediär gebildete Verbindung III bzw. ein Tautomeres und/oder Salz davon im allgemeinen ohne Isolierung erfindungsgemäss weiterreagiert.

In Gruppen der Formel IIIb steht $X_3$ für gegebenenfalls funktionell abgewandeltes Oxo, wie Oxo, Thioxo oder gegebenenfalls substituiertes Imino, wie Imino, N-Niederalkylimino, N-Cycloalkylimino oder N-Niederalkanoylimino, ferner gegebenenfalls substituiertes N-Benzoylimino, N-Niederalkansulfonylimino oder N-Arylimino.

Die Cyclisierung von Verbindungen IIIa, deren Tautomeren und jeweils ihren Salzen und gegebenenfalls die nachfolgende in situ-Eliminierung von Y-H aus den gebildeten Verbindungen III bzw. deren Tautomeren

und/oder Salzen erfolgen in üblicher Weise, z.B. unter neutralen, sauren oder basischen Bedingungen, erforderlichenfalls in Gegenwart einer Säure oder eines basischen Mittels, in Gegenwart eines inerten Lösungs- oder Verdünnungsmittels, bei Raumtemperatur oder vorzugsweise unter Erwärmen, z.B. im Temperaturbereich von etwa 20 bis etwa 250° C, und/oder unter Inertgas, wie Stickstoff. Als Säuren, basische Mittel und inerte Lösungs-oder Verdünnungsmittel können beispielsweise die entsprechenden unter der Verfahrensvariante a) erläuterten Agentien Verwendung finden. Als inertes Lösungs- oder Verdünnungsmittel kann in besonders vorteilhafter Weise aber auch ein Niederalkansäureamid, wie Acetamid, dienen.

In einer besonders bevorzugten Ausführungsform wird beispielsweise eine Verbindung IIIa, worin $Y_5$ und $Y_7$ Wasserstoff und $Y_6$ eine Gruppe $R_2$-C(=O)-NH- (IIIb) bedeuten oder $Y_5$ und $Y_6$ gemeinsam für eine zusätzliche Bindung und $Y_7$ für eine Gruppe $R_2$-C(OH)($NH_2$)- (IIIC) stehen, oder ein Tautomeres und/oder Salz davon durch mehrstündiges Erhitzen, beispielsweise im Temperaturbereich von etwa 80 bis etwa 200° C, in einem Niederalkansäureamid, wie Acetamid, cyclisiert, wobei unter den Reaktionsbedingungen in situ eine Weiterreaktion der gebildeten Verbindung III, worin Y Hydroxy ist, bzw. eines Tautomeren und/oder Salzes davon zu dem gewünschten Endprodukt der Formel I bzw. einem Salz davon eintritt. In analoger Weise lassen sich entsprechende Verbindungen IIIa mit Gruppen IIIb, worin $X_3$ Imino ist, oder Gruppen IIIc, worin Y Amino ist, oder Tautomere und/oder Salze davon in einem inerten Lösungsmittel, wie einem Halogenalkan, beispielsweise Tetrachlormethan, cyclisieren und zu Verbindungen I bzw. deren Salzen weiterumsetzen.

Die Verbindungen IIIa, deren Tautomere und jeweils ihre Salze erhält man aus Verbindungen der Formel

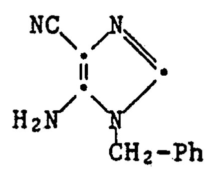

(IIId)

bzw. deren Salzen durch Reaktion mit einer Verbindung der Formel $R_2$-C(=$X_3$)-$NH_2$ (IIIe), worin $X_3$ für gegebenenfalls funktionell abgewandeltes Oxo, beispielsweise der bei der Gruppe IIIb erläuterten Art, steht, bzw. deren Salzen, wobei in Analogie zu bekannten Verfahren gearbeitet wird, beispielsweise bei Raumtemperatur oder vorzugsweise unter Erwärmen, z.B. im Temperaturbereich von etwa 20 bis etwa 250° C, unter neutralen, sauren oder basischen Bedingungen, erforderlichenfalls in Gegenwart einer Säure oder eines basischen Mittels, beispielweise der vorstehend erwähnten Art, in Gegenwart eines inerten Lösungs- oder Verdünnungsmittels, z.B. der vorstehend erwähnten Art, und/oder unter Inertgas, wie Stickstoff. Als inertes Lösungs- oder Verdünnungsmittel kann dabei insbesondere auch ein Niederalkansäureamid, wie Acetamid, dienen.

In einer besonders bevorzugten Ausführungsform wird eine Verbindung IIId bzw. ein Salz davon mit einem grossen Ueberschuss einer Verbindung IIIe ($X_3$ = gegebenenfalls funktionell abgewandeltes Oxo, vorzugsweise Oxo), beispielsweise in Acetamid ($X_3$ = Oxo, $R_2$ = Methyl), unter Erwärmen, beispielsweise im Temperaturbereich von etwa 80 bis etwa 200° C, zu einer Verbindung IIIa bzw. einem Tautomeren und/oder Salz davon umgesetzt, wobei die Reaktionskomponente IIIe gleichzeitig als Lösungs- bzw. Verdünnungsmittel dient.

Vorteilhafterweise werden allerdings auch die Verbindungen IIIa, deren Tautomere und jeweils ihre Salze in situ hergestellt und ohne Isolierung zu Verbindungen III bzw. deren Tautomeren und/oder Salzen weiterumgesetzt, welche ihrerseits, wie vorstehend beschrieben, im allgemeinen gleichfalls in situ zu Verbindungen I bzw. deren Salzen weiterreagieren.

So kann nach Art einer Eintopfreaktion eine Verbindung IIId bzw. ein Salz davon unter Erwärmen mit einem grossen Ueberschuss einer Verbindung IIIe ($X_3$ = gegebenenfalls funktionell abgewandeltes Oxo,vorzugsweise Oxo), beispielsweise in Acetamid ($X_3$ = Oxo, $R_2$ = Methyl), umgesetzt werden, wobei zunächst eine Verbindung IIIa bzw. ein Tautomeres und/oder Salz davon gebildet wird, woran sich in situ bei Aufrechterhaltung der Wärmezufuhr die Cyclisierung zu einer Verbindung III bzw. einem Tautomeren und/oder Salz davon anschliesst, ihrerseits gefolgt von einer in situ-Eliminierung einer Verbindung Y-H (Y = Hydroxy) zu einer Verbindung I ($R_1$ = Amino, $R_2$ beispielsweise = Methyl) bzw. einem Salz davon.

Die Cyclisierung von in der Verfahrensvariante c) verwendeten Ausgangsstoffen IV, deren Tautomeren und jeweils ihren Salzen zu Verbindungen I, worin $R_2$ Amino bedeutet, bzw. deren Salzen erfolgt unter den üblichen Cyclisierungsbedingungen, beispielsweise in analoger Weise wie unter der Verfahrensvariante b)

für Cyclisierungen von Verbindungen IIIa zu Verbindungen III beschrieben.

Die Ausgangsstoffe IV, deren Tautomere und jeweils ihre Salze lassen sich in Analogie zu bekannten Methoden erhalten, beispielsweise durch Reaktion einer Verbindung der Formel

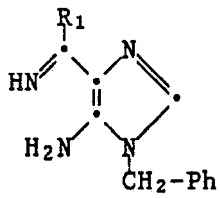

(IVa)

oder eines Tautomeren und/oder Salzes davon mit einer Verbindung der Formel $X_1$-C≡N (IVb), worin $X_1$ eine nucleofuge Abgangsgruppe, beispielsweise der unter der Verfahrensvariante a) für Gruppen $X_1$ beschriebenen Art, vorzugsweise Halogen, wie Chlor oder Brom, oder freies oder wie für Reste $R_1$ bzw. $R_2$ angegeben substituiertes Amino darstellt, wobei unter den üblichen Reaktionsbedingungen, beispielsweise bei Raumtemperatur oder unter Erwärmen, in einem inerten Lösungs- oder Verdünnungsmittel, z.B. der vorstehend erwähnten Art, gegebenenfalls in Gegenwart eines Kondensationsmittels, beispielsweise eines basischen Mittels, z.B. der vorstehend beschriebenen Art, und/oder unter Inertgas, wie Stickstoff, gearbeitet wird.

In einer bevorzugten Ausführungsform werden die Verbindungen IV bzw. deren Tautomere und/oder Salze nicht isoliert, sondern in situ generiert und ohne Isolierung oder zusätzliche Reinigung erfindungsgemäss zu Verbindungen I bzw. deren Salzen cyclisiert.

Die Verbindungen IVa, deren Tautomere und jeweils ihre Salze können in Analogie zu bekannten Methoden hergestellt werden, beispielsweise durch Umsetzung einer Verbindung IIId bzw. eines Salzes davon mit einer Verbindung der Formel H-$R_1$ (IIb) bzw. einem Salz davon unter den üblichen Reaktionsbedingungen.

Reaktionsfähige Derivate der Ameisensäure, die als Ausgangsstoffe gemäss der Verfahrensvariante d) verwendet werden können, sind beispielsweise Verbindungen der Formel HC($Y_1$)($Y_2$)($Y_3$) (Va), worin $Y_1$ und $Y_2$ gemeinsam gegebenenfalls funktionell abgewandeltes Oxo, z.B. Oxo, Thioxo oder gegebenenfalls substituiertes Imino, wie Imino, N-Niederalkylimino, N-Cycloalkylimino, N-Niederalkanoylimino, gegebenenfalls substituiertes N-Benzoylimino, N-Niederalkansulfonylimino oder N-Arylimino, bedeuten und $Y_3$ Niederalkoxy, wie Methoxy, Niederalkylthio, wie Methylthio, Mercapto, Halogen, wie Chlor oder Brom, oder gegebenenfalls substituiertes Amino, wie Amino, N-Niederalkylamino oder N,N-Diniederalkylamino, darstellt oder worin $Y_1$, $Y_2$ und $Y_3$ jeweils Niederalkoxy, wie Methoxy oder Aethoxy, oder Niederalkylthio, wie Methylthio, bedeuten, wobei Verbindungen Va, worin $Y_1$, $Y_2$ und $Y_3$ jeweils Methoxy oder Aethoxy bedeuten, also Trimethyl- und Triäthylorthoformiat, besonders bevorzugt sind.

Die Umsetzung einer Verbindung V oder eines Salzes davon mit Ameisensäure oder einem reaktionsfähigen Derivat davon erfolgt in Analogie zu bekannten Verfahrensweisen unter den üblichen Reaktionsbedingungen, beispielsweise in einem inerten Lösungs- oder Verdünnungsmittel oder in einem Gemisch derselben, bei Raumtemperatur oder unter Erwärmen, beispielsweise im Temperaturbereich von etwa +20 bis etwa +150 °C, vorzugsweise von etwa +20 bis etwa +100 °C, gegebenenfalls in Gegenwart eines sauren Mittels, z.B. einer der unter der Verfahrensvariante b) angegebenen Säuren, insbesondere einer organischen Sulfonsäure, und/oder unter Inertgas, wie Stickstoff.

Als inerte Lösungs- oder Verdünnungsmittel kommen insbesondere die unter der Verfahrensvariante a) angegebenen Mittel in Betracht, vorteilhaft kann jedoch auch in einem Ueberschuss der Ameisensäurekomponente, z.B. in einem Ueberschuss von Trimethyl- oder Triäthylorthoformiat, gearbeitet werden.

Die Ausgangsstoffe V und deren Salze sind bekannt oder lassen sich in Analogie zu bekannten Methoden, beispielsweise durch Umsetzung einer Verbindung der Formel

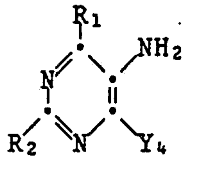

(Vb),

worin $Y_4$ für eine nucleofuge Abgangsgruppe, z.B. eine Gruppe $Z_1$ gemäss der Verfahrensvariante a), wie

Halogen, steht, oder eines Salzes davon mit einer Verbindung der Formel Ph-$CH_2$-$NH_2$ oder einem Salz davon, herstellen, wobei unter den üblicherweise angewendeten Reaktionsbedingungen, beispielsweise wie unter der Verfahrensvariante a) angegeben, gearbeitet wird.

Die Derivate Va und die Verbindungen Vb und deren Salze sind bekannt oder in Analogie zu bekannten Methoden herstellbar.

Salze der in der Verfahrensvariante e) verwendeten Ausgangsstoffe VI sind insbesondere Metallsalze, wie Alkali- oder Erdalkalimetallsalze, beispielsweise Natrium-, Kalium-, Magnesium- oder Calciumsalze, oder Uebergangsmetallsalze, z.B. pharmazeutisch verwendbare Uebergangsmetallsalze, beispielsweise Zink- oder Kupfersalze, derselben.

Nucleofuge Abgangsgruppen $X_1$ in Verbindungen VII sind beispielsweise solche der unter der Verfahrensvariante a) für Gruppen $X_1$ angegebenen Art.

Die Umsetzung einer Verbindung VI mit einer Verbindung VII erfolgt in üblicher Weise, beispielsweise in Gegenwart eines basischen Kondensationsmittels oder vorteilhaft, indem man die Komponente der Formel VI in Form eines ihrer Metallsalze einsetzt, bei Raumtemperatur oder vorzugsweise unter Erwärmen, beispielsweise in einem Temperaturbereich von etwa 20 bis etwa 200 °C, insbesondere von etwa 50 bis etwa 150 °C, in einem inerten Lösungs- oder Verdünnungsmittel, beispielsweise der vorstehend erwähnten Art, gegebenenfalls in Gegenwart eines Phasentransfer-Katalysators, z.B. eines quaternären Ammoniumsalzes, wie Tetrabutylammoniumbromid oder Benzyltrimethylammoniumchlorid, oder quaternären Phosphoniumsalzes, und/oder unter Inertgas, wie Stickstoff. Als basische Kondensationsmittel sind insbesondere mit der Komponente VI salzbildende basische Kondensationsmittel geeignet, beispielsweise die unter der Verfahrensvariante a) erwähnten basischen Mittel. Wie erwähnt wird die Ueberführung der Komponente VI in eines ihrer Salze vorteilhaft vorab durchgeführt, beispielsweise durch Umsetzung mit einem der genannten basischen Mittel. Besonders geeignet sind Ausgangsstoffe VI und deren Salze, worin $R_1$ und $R_2$ von Amino und wie angegeben monosubstituiertem Amino verschieden sind.

Die Ausgangsstoffe VI sind bekannt oder in Analogie zu bekannten Methoden herstellbar, beispielsweise analog der Verfahrensvariante a) ausgehend von entsprechenden Verbindungen II oder deren Tautomeren und/oder Salzen, die in 9-Position anstelle des Ph-$CH_2$-Substituenten ein Wasserstoffatom aufweisen. Die Ausgangsstoffe VII sind bekannt oder können in Analogie zu bekannten Methoden erhalten werden.

Geeignete Aminoschutzgruppen $S_1$ bzw. $S_2$ in einer gemäss der Verfahrensvariante f) als Ausgangsstoff verwendeten Verbindung VIII oder einem Salz davon sowie Verfahren zu deren Einführung und Abspaltung sind aus dem Stand der Technik, der bis in die Einzelheiten ausgearbeitet ist, insbesondere als allgemeine Methoden für die Synthese von Peptiden, bekannt [vgl. z.B. Houben-Weyl: Methoden der Organischen Chemie, 4. Ausgabe, Band 15/I und II, E. Wünsch (Herausgeber): Synthese von Peptiden (Georg Thieme-Verlag, Stuttgart, 1974)]. Geeignet sind insbesondere Aminoschutzgruppen vom Benzyloxycarbonyl-Typ, bei denen die Benzyloxycarbonylgruppe an dem aromatischen Teil durch Halogen, Niederalkoxy und/oder Niederalkyl und insbesondere durch Nitro substituiert sein kann, wie die p-Chlor- und p-Brombenzyloxycarbonyl-, p-Methoxybenzyloxycarbonyl-, p-Methylbenzyloxycarbonyl- und insbesondere p-Nitrobenzyloxycarbonylgruppe, oder alternativ die Isonicotinyloxycarbonylgruppe, weiterhin Aethoxycarbonylgruppen, die in der β-Stellung eine durch drei Kohlenwasserstoffreste substituierte Silylgruppe enthalten, wie Triphenylsilyl, Dimethyl-tert.-butylsilyl oder insbesondere Trimethylsilyl, z.B. eine β-(Triniederalkylsilyl)-äthoxycarbonyl-, wie β-(Trimethylsilyl)äthoxycarbonylgruppe, sowie tert.-Butoxycarbonylgruppen und analoge Gruppen, ebenso diejenigen des Aralkyl-Typs, wie Benzhydryl, Di-(4-methoxy)benzhydryl und Triphenylmethyl (Trityl), oder bestimmte Aralkoxycarbonylgruppen vom 2-(p-Biphenylyl)-2-propoxycarbonyl-Typ, die in der CH-PS 509 266 beschrieben werden.

Die Ueberführung von $S_1$ in $R_1$ und/oder $S_2$ in $R_2$, d.h. die Abspaltung der Aminoschutzgruppe(n) $S_1$ und/oder $S_2$, erfolgt in der üblichen, z.B. in der erwähnten Literatur ausführlich behandelten, Weise, beispielsweise durch Solvolyse, wie milde Hydrolyse, z.B. Behandlung mit Wasser unter neutralen oder schwach sauren Bedingungen, z.B. durch Einwirkung von verdünnt-wässrigen Mineral- oder Carbonsäuren, z.B. von verdünnter Salz- oder Essigsäure, durch Einwirkung von Fluoridionen, z.B. mittels eines Alkalimetallfluorides, oder durch Reduktion, z.B. entsprechend den unter der Verfahrensvariante a) angegebenen Reduktionsbedingungen.

Die Verbindungen VIII und deren Salze können z.B. analog den Verfahrensvarianten a) bis e) erhalten werden, indem man von in üblicher Weise erhältlichen Ausgangsstoffen II, IIb, IIj, III, IV, V und VI ausgeht, welche anstelle der Aminogruppen $R_1$ bzw. $R_2$ eine Aminoschutzgruppe $S_1$ bzw. $S_2$ aufweisen.

Falls erforderlich, können bei Durchführung der verfahrensgemässen Reaktionen gemäss den Verfahrensvarianten a) bis e) Aminogruppen $R_1$ und/oder $R_2$ auch intermediär geschützt vorliegen, z.B. als Aminoschutzgruppen $S_1$ und/oder $S_2$. Die Einführung und Abspaltung solcher Gruppen $S_1$ und/oder $S_2$ erfolgt z.B. entsprechend den unter der Verfahrensvariante f) angegebenen Bedingungen.

Gegebenenfalls kann dabei eine verfahrensgemässe Reaktion auch mit der Abspaltung der Aminoschutzgruppe(n) $S_1$ und/oder $S_2$ kombiniert werden, z.B. kann die Ueberführung einer gegebenenfalls verätherten Mercaptogruppe $X_1$ in Wasserstoff $R_1$ gemäss der Verfahrensvariante a) mit der Abspaltung einer Aminoschutzgruppe $S_2$ gemäss der Verfahrensvariante f) kombiniert werden.

Verfahrensgemäss oder anderweitig erhältliche Verbindungen der Formel I können in andere Verbindungen der Formel I überführt werden, indem man eine oder mehrere Variablen der allgemeinen Formel I in andere überführt.

So kann man in Verbindungen I unsubstituiertes Amino $R_1$ und/oder $R_2$ in N-Mono- oder N,N-Diniederalkylamino, ebenso N-Mononiederalkylamino $R_1$ und/oder $R_2$ in N,N-Diniederalkylamino überführen, beispielsweise durch Behandeln mit einem reaktiven Ester eines Niederalkanols, wie einem Niederalkylhalogenid, z.B. -bromid oder -iodid, Niederalkansulfonat, z.B. Methansulfonat, einem gegebenenfalls substituierten Arylsulfonat, wie Benzol- oder p-Toluolsulfonat, oder einem Diniederalkylsulfat, z.B. Dimethylsulfat, vorzugsweise unter basischen Bedingungen, wie in Gegenwart von Natriumhydrid oder von Natronlauge oder Kalilauge und vorteilhaft in Gegenwart eines Phasentransfer-Katalysators, wie Tetrabutylammoniumbromid oder Benzyltrimethylammoniumchlorid. Dabei kann entweder nur eine N-Niederalkylgruppe eingeführt werden, oder es können in einem einzigen Reaktionsschritt auch mehrere, insbesondere 2 bis und mit 4, N-Niederalkylgruppen eingeführt werden. Ebenso ist es möglich, in aufeinanderfolgenden Reaktionsschritten bei geeigneter Wahl der Niederalkylkomponenten unterschiedliche N-Niederalkylgruppen in unsubstituiertes Amino bzw. N-Mononiederalkylamino $R_1$ und/oder $R_2$ einzuführen. Gemäss dieser Methode der N-Alkylierung erhält man jeweils Verbindungen I, in denen alle im selben Reaktionsschritt eingeführten N-Niederalkylgruppen gleichartig sind.

In analoger Weise kann auch in N-(Hydroxyniederalkyl)amino, N-Monocycloalkylamino, N-Mono-(cycloalkylniederalkyl)amino und N-Niederalkanoylamino $R_1$ und/oder $R_2$ jeweils eine N-Niederalkylgruppe eingeführt werden, wobei man zu N-(Hydroxyniederalkyl)-N-niederalkyl-amino, N-Cycloalkyl-N-niederalkylamino, N-(Cycloalkylniederalkyl)-N-niederalkylamino und N-Niederalkanoyl-N-niederalkyl-amino $R_1$ und/oder $R_2$ gelangt.

Ebenso kann bei entsprechender sinnvoller Abwandlung der Alkylierungskomponenten auch unsubstituiertes Amino $R_1$ und/oder $R_2$ durch Einführung einer N-(Niederalkoxyniederalkyl)gruppe in N-(Niederalkoxyniederalkyl)amino $R_1$ und/oder $R_2$, durch Einführung einer N-(Hydroxyniederalkyl)gruppe in N-(Hydroxyniederalkyl)amino $R_1$ und/oder $R_2$, durch Einführung einer oder mehrerer, insbesondere 2 bis und mit 4, N-Cycloalkylgruppe(n) in N-Mono- oder N,N-Dicycloalkylamino $R_1$ und/oder $R_2$ oder durch Einführung einer oder mehrerer, insbesondere 2 bis und mit 4, N-(Cycloalkylniederalkyl)gruppe(n) in N-Mono- oder N,N-Di(cycloalkylniederalkyl)amino $R_1$ und/oder $R_2$, weiterhin N-Niederalkylamino $R_1$ und/oder $R_2$ durch Einführung einer N-(Hydroxyniederalkyl)gruppe in N-(Hydroxyniederalkyl)-N-niederalkyl-amino $R_1$ und/oder $R_2$, durch Einführung einer N-Cycloalkylgruppe in N-Cycloalkyl-N-niederalkyl-amino $R_1$ und/oder $R_2$ oder durch Einführung einer N-(Cycloalkylniederalkyl)gruppe in N-(Cycloalkylniederalkyl)-N-niederalkylamino $R_1$ und/oder $R_2$, weiterhin N-Monocycloalkylamino $R_1$ und/oder $R_2$ durch Einführung einer N-Cycloalkylgruppe in N,N-Dicycloalkylamino $R_1$ und/oder $R_2$ sowie N-Mono(cycloalkylniederalkyl)amino $R_1$ und/oder $R_2$ durch Einführung einer N-(Cycloalkylniederalkyl)gruppe in N,N-Di(cycloalkylniederalkyl)amino $R_1$ und/oder $R_2$ umgewandelt werden.

Weiterhin kann man unsubstituiertes Amino $R_1$ und/oder $R_2$ N-acylieren, beispielsweise durch Umsetzung mit einer Niederalkansäure, wie Ameisen-, Essig- oder Propionsäure, oder einem reaktiven Derivat einer solchen Säure, z.B. einem Säurehalogenid, wie Säurechlorid, Ester oder insbesondere einem Anhydrid, z.B. Acetylchlorid oder Acetanhydrid, in N-Niederalkanoylamino $R_1$ und/oder $R_2$ überführen. Ebenso kann N-Niederalkylamino $R_1$ und/oder $R_2$ in N-Niederalkanoyl-N-niederalkyl-amino $R_1$ und/oder $R_2$ überführt werden. Dabei ist es wiederum möglich, entweder nur eine N-Acylgruppe einzuführen oder in einem Reaktionsschritt sowohl Amino oder N-Niederalkylamino $R_1$ als auch Amino oder N-Niederalkylamino $R_2$ zu N-acylieren. Ebenso ist es möglich, in aufeinanderfolgenden Reaktionsschritten bei geeigneter Wahl der Acylierungsagentien unterschiedliche N-Acylgruppen in unsubstituiertes Amino oder N-Niederalkylamino $R_1$ und $R_2$ einzuführen. In jedem Fall erhält man jeweils Verbindungen I, in denen alle im selben Reaktionsschritt eingeführten N-Acylgruppen gleichartig sind.

Ebenso lässt sich N-Niederalkanoylamino $R_1$ und/oder $R_2$ in unsubstituiertes Amino $R_1$ und/oder $R_2$ überführen, beispielsweise durch Reduktion, also Austausch der Acylgruppe(n) gegen Wasserstoff, wozu die üblichen Reduktionssysteme und Reaktionsbedingungen in Betracht kommen, z.B. Diboran, Lithiumaluminiumhydrid in Tetrahydrofuran, Diäthyläther oder Dioxan, Natriumborhydrid/Cobalt(II)chlorid, Natriumborhydrid/Trifluoressigsäure oder Trihalogensilane, wie Trichlorsilan. Weiterhin lässt sich N-Niederalkanoylamino $R_1$ und/oder $R_2$ auch durch Hydrolyse in unsubstituiertes Amino $R_1$ und/oder $R_2$ überführen, wobei unter den üblichen Reaktionsbedingungen gearbeitet wird, beispielsweise in wässriger Lösung, in

Gegenwart eines basischen Mittels, insbesondere z.B. in Gegenwart eines Alkalimetallhydroxids oder -niederalkanolats, wie Natrium- oder Kaliumhydroxid oder Natriummethanolat, vorzugsweise in einem organischen Lösungs- oder Verdünnungsmittel bzw. Cosolvens und/oder unter Erwärmen, vorzugsweise im Temperaturbereich von etwa 20 bis etwa 150° C, insbesondere von etwa 40 bis etwa 100° C. Dabei ist es möglich, je nach Anzahl eingesetzter Aequivalente des Reduktionsmittels bzw. des basischen Mittels nur eine oder, falls vorhanden, beide Acylgruppen zu unsubstituiertem Amino $R_1$ bzw. $R_2$ zu reduzieren bzw. hydrolysieren.

Weiterhin lässt sich N-Mononiederalkylamino $R_1$ und/oder $R_2$ in unsubstituiertes Amino $R_1$ und/oder $R_2$ überführen, indem zunächst die N-Niederalkylgruppe in üblicher Weise zur N-Niederalkanoylgruppe oxidiert wird und letztere dann wie angegeben in die unsubstituierte Aminogruppe umgewandelt wird.

Halogen oder Niederalkoxy $R_2$ kann in analoger Weise wie unter der Verfahrensvariante a) für die Umwandlung von Resten $Z_1$ bzw. $Z_2$ in $R_1$ bzw. $R_2$ beschrieben in andere Reste $R_2$ überführt werden. Ebenso kann Halogen $R_2$ auch gegen Niederalkoxy $R_2$ ausgetauscht werden.

Die neuen Verbindungen der Formel I und ihre Salze können je nach Anzahl der asymmetrischen Kohlenstoffatome Stereoisomere, beispielsweise Diastereomere oder Enantiomere, bilden. Asymmetrische Kohlenstoffatome können beispielsweise in den Verbindungen I bzw. deren Salzen in entsprechenden Niederalkylresten $R_2$ auftreten.

Erhaltene Isomeren- und Diastereomerengemische können auf Grund der unterschiedlichen physikalischen Eigenschaften der Komponenten nach üblichen physikalischen Trennverfahren, beispielsweise durch Destillation, Kristallisation und/oder Chromatographie, in die Komponenten aufgetrennt werden.

Erhaltene Enantiomerengemische, z.B. Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man jeweils das wirksamere Stereoisomere.

Ferner können erhaltene freie Verbindungen der Formel I mit basischen Zentren in an sich bekannter Weise in Säureadditionssalze überführt werden, z.B. durch Umsetzen einer Lösung der freien Verbindung in einem geeigneten Lösungsmittel oder Lösungsmittelgemisch mit einer der vorgenannten Säuren oder mit einer Lösung davon oder mit einem geeigneten Ionenaustauscher.

Erhaltene Säureadditionssalze von Verbindungen der Formel I können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder mit einem geeigneten Ionenaustauscher.

Erhaltene Säureadditionssalze von Verbindungen der Formel I können in an sich bekannter Weise in andere Säureadditionssalze überführt werden, z.B. durch Behandlung eines Salzes einer organischen Säure mit einem geeigneten Metallsalz, wie einem Natrium-, Barium- oder Silbersalz, einer Säure in einem geeigneten Lösungsmittel, in welchem ein sich bildendes anorganisches Salz unlöslich ist und damit aus dem Reaktionsgemisch ausscheidet.

Die neuen Verbindungen der Formel I und ihre Salze können auch in Form ihrer Hydrate erhalten werden und/oder andere, z.B. gegebenenfalls zur Kristallisation von in fester Form vorliegenden Stoffen verwendete, Lösungsmittel einschliessen.

Je nach Verfahrensweise bzw. Reaktionsbedingungen können die erfindungsgemässen Verbindungen der Formel I in freier Form oder in Form ihrer Salze erhalten werden.

Infolge der engen Beziehung zwischen den neuen Verbindungen der Formel I in freier Form und in Form ihrer Salze sind vorstehend und nachfolgend unter den freien Verbindungen der Formel I sinn- und zweckgemäss gegebenenfalls auch die entsprechenden Salze bzw. unter den Salzen auch die entsprechenden freien Verbindungen der Formel I zu verstehen.

Die Erfindung betrifft auch diejenigen Ausführungsformen des Verfahrens, nach denen man von einer der auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Neue Ausgangsstoffe, die speziell für die Herstellung der erfindungsgemässen Verbindungen entwickelt wurden, insbesondere die zu den eingangs als bevorzugt gekennzeichneten Verbindungen der Formel I

führende Ausgangsstoffauswahl, die Verfahren zu ihrer Herstellung sowie ihre Verwendung als Zwischenprodukte bilden ebenfalls einen Gegenstand der Erfindung.

Die Erfindung betrifft ebenfalls die Verwendung der neuen Verbindungen der Formel I bzw. ihrer pharmazeutisch verwendbaren Salze, insbesondere als pharmakologische, in erster Linie antikonvulsiv wirksame, Wirksubstanzen. Dabei kann man sie, vorzugsweise in Form pharmazeutischer Präparate, in einem Verfahren zur prophylaktischen und/oder therapeutischen Behandlung des tierischen oder menschlichen Körpers, insbesondere als Antikonvulsiva, z.B. zur Behandlung von Konvulsionen verschiedenartiger Genese, beispielsweise zur Behandlung der Epilepsie, anwenden.

Die Erfindung betrifft gleichfalls pharmazeutische Präparate, die eine Verbindung der Formel I bzw. ein pharmazeutisch verwendbares Salz davon als Wirkstoff enthalten, sowie Verfahren zu ihrer Herstellung.

Bei den erfindungsgemässen pharmazeutischen Präparaten handelt es sich um solche, die eine therapeutisch wirksame Menge der erfindungsgemässen Aktivsubstanz, gegebenenfalls zusammen mit anorganischen oder organischen, festen oder flüssigen, pharmazeutisch verwendbaren Hilfsstoffen enthalten, und die sich zur enteralen, z.B. oralen, oder parenteralen Verabreichung an Warmblüter eignen. So verwendet man vorzugsweise pharmazeutische Präparate in Dosiseinheitsformen, wie Dragees, Tabletten, Kapseln oder Suppositorien, ferner Ampullen, welche den Wirkstoff zusammen mit Verdünnungsmitteln, z.B. Laktose, Dextrose, Saccharose, Mannitol, Sorbitol, Cellulose und/oder Glycin, und/oder Schmiermitteln, z.B. Kieselerde, Talk, Stearinsäure oder Salze davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, Adsorp tionsmittel, Farbstoffe, Geschmacksstoffe und/oder Süssmittel enthalten. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer, enthalten. Die neuen pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis etwa 100 %, insbesondere von etwa 1 % bis etwa 50 %, Lyophilisate bis zu 100 % des Aktivstoffes.

Die Dosierung kann von verschiedenen Faktoren, wie Applikationsweise, Warmblüter-Spezies, Alter und/oder individuellem Zustand abhängen. Die täglich zu verabreichenden Dosen liegen im Normalfall bei oraler Applikation zwischen etwa 1 und etwa 30 mg/kg und für Warmblüter mit einem Gewicht von etwa 70 kg vorzugsweise zwischen etwa 0,1 g und etwa 3,0 g, wobei zur Erreichung der Tagesdosis auch mehrere Teildosen verabreicht werden können.

Auch die bekannten Verbindungen der Formel I, die von den vorstehend erwähnten Massgaben umfasst sind, können in freier Form oder in Form pharmazeutisch verwendbarer Salze in analoger Weise wie vorstehend erläutert als pharmakologische, insbesondere antikonvulsiv wirksame, Wirksubstanzen verwendet werden, z.B., vorzugsweise in Form der vorstehend erläuterten pharmazeutischen Präparate, in einem der vorstehend erläuterten Behandlungsverfahren; die entsprechenden Verwendungen, Behandlungsverfahren, pharmazeutischen Präparate und Herstellungsverfahren für letztere bilden ebenfalls einen Gegenstand der Erfindung.

Die folgenden Beispiele dienen zur Illustration der oben beschriebenen Erfindung; sie sollen jedoch diese in ihrem Umfang in keiner Weise einschränken. Temperaturen werden in Celsiusgraden angegeben.

Beispiel 1:

3,31 g (16,8 mmol) 2-Chlor-6-(N,N-dimethylamino)-9H-purin werden in 60 ml N,N-Dimethylformamid gelöst. Die Lösung wird mit 4,16 g (30,2 mmol) Kaliumcarbonat und 2,02 ml (16,8 mmol) 2-Fluorbenzylbromid versetzt. Das Reaktionsgemisch wird sodann 3 Stunden bei Raumtemperatur gerührt und anschliessend mit Eiswasser versetzt und dreimal mit Dichlormethan extrahiert. Die vereinigten organischen Phasen werden zweimal mit Wasser gewaschen, über Magnesiumsulfat getrocknet und eingedampft. Der Rückstand wird an Kieselgel mit Toluol/Aethylacetat (3:1) als Laufmittel chromatographiert. Das Eluat wird eingedampft und der erhaltene Rückstand aus Aethylacetat/Diäthyläther umkristallisiert. Man erhält auf diese Weise das

2-Chlor-6-(N,N-dimethylamino)-9-(2-fluorbenzyl)-9H-purin in Form von farblosen Nadeln, die einen Schmelzbereich von 130 bis 131° aufweisen.

Das 2-Chlor-6-(N,N-dimethylamino)-9H-purin kann man z.B. folgendermassen erhalten: Xanthin (2,6-Dihydroxy-9H-purin) wird durch Umsetzung mit Phosphoroxytrichlorid gemäss G.B. Elion u. G.H. Hitchings, J. Am. Chem. Soc. 78, 3508 (1956) in das 2,6-Dichlor-9H-purin überführt und letzteres mit N,N-Dimethylamin gemäss J.A. Montgomery u. L.B. Holum, J. Am. Chem. Soc. 80, 404 (1958) zu 2-Chlor-6-(N,N-dimethylamino)-9H-purin umgesetzt.

Beispiel 2:

2,5 g (8,2 mmol) 2-Chlor-6-(N,N-dimethylamino)-9-(2-fluorbenzyl)-9H-purin (Beispiel 1) und 10 ml äthanolische N-Methylaminlösung (16 %) werden in einem Autoklaven 120 Stunden auf 80 bis 85° erwärmt. Anschliessend wird das Reaktionsgemisch unter vermindertem Druck eingedampft. Der Rückstand wird in Dichlormethan aufgenommen. Die Dichlormethanphase wird zweimal mit Wasser gewaschen, über Magnesiumsulfat getrocknet und eingedampft. Der Rückstand wird an Kieselgel mit Toluol/Aethylacetat (10:1) als Laufmittel chromatographiert. Das Eluat wird eingedampft und der erhaltene Rückstand aus Dichlormethan/Hexan umkristallisiert. Man erhält so das 6-(N,N-Dimethylamino)-9-(2-fluorbenzyl)-2-(N-methylamino)-9H-purin in Form von farblosen Nadeln vom Schmelzpunkt 100°.

Beispiel 3:

In analoger Weise wie in Beispiel 2 beschrieben kann man unter Verwendung von Ammoniak anstelle von N-Methylamin auch das 2-Amino-6-(N,N-dimethylamino)-9-(2-fluorbenzyl)-9H-purin, das einen Schmelzbereich von 197 bis 198° (Methanol/Diäthyläther) aufweist, erhalten, wobei das Reaktionsgemisch 48 Stunden auf 160° erwärmt wird.

Beispiel 4:

In analoger Weise wie in Beispiel 2 beschrieben kann man unter Verwendung von N-Aethylamin anstelle von N-Methylamin auch das 2-(N-Aethylamino)-6-(N,N-dimethylamino)-9-(2-fluorbenzyl)-9H-purin, das einen Schmelzbereich von 76 bis 77° (Dichlormethan/Hexan) aufweist, erhalten, wobei das Reaktionsgemisch 160 Stunden auf 90 bis 95° erwärmt wird.

Beispiel 5:

In analoger Weise wie in Beispiel 2 beschrieben kann man unter Verwendung von N,N-Dimethylamin anstelle von N-Methylamin auch das 2,6-Bis(N,N-dimethylamino)-9-(2-fluorbenzyl)-9H-purin, das einen Schmelzpunkt von 127° (Dichlormethan/Diäthyläther) aufweist, erhalten, wobei das Reaktionsgemisch 24 Stunden auf 140° erwärmt wird.

Beispiel 6:

In analoger Weise wie in Beispiel 1 beschrieben kann man unter Verwendung von 2-Chlor-6-(N-methylamino)-9H-purin anstelle von 2-Chlor-6-(N,N-dimethylamino)-9H-purin auch das 2-Chlor-9-(2-fluorbenzyl)-6-(N-methylamino)-9H-purin, das einen Schmelzbereich von 169 bis 170° (Methanol/Diäthyläther) aufweist, erhalten.

Das 2-Chlor-6-(N-methylamino)-9H-purin kann man z.B. erhalten, indem man 2,6-Dichlor-9H-purin in analoger Weise wie in Beispiel 1 beschrieben mit N-Methylamin umsetzt.

Beispiel 7:

6,5 g (25,7 mmol) 5-Amino-2-chlor-4-[N-(2-fluorbenzyl)amino]-pyrimidin werden bei Raumtemperatur in

65 ml Triäthylorthoformiat gelöst. Die Lösung wird mit 30 mg Methansulfonsäure versetzt und das Reaktionsgemisch dann 15 Stunden bei Raumtemperatur gerührt. Anschliessend wird das Reaktionsgemisch eingedampft und der Rückstand an Kieselgel mit Toluol/Aethylacetat (3:1) als Laufmittel chromatographiert. Das Eluat wird eingedampft und der erhaltene Rückstand aus Dichlormethan/Diäthyläther umkristallisiert. Man erhält so das 2-Chlor-9-(2-fluorbenzyl)-9H-purin in Form von farblosen Kristallen, die einen Schmelzbereich von 103 bis 104° aufweisen.

Das 5-Amino-2-chlor-4-[N-(2-fluorbenzyl)amino]-pyrimidin kann z.B. wie folgt erhalten werden: Ein Gemisch von 0,5 g (3,1 mmol) 5-Amino-2,4-dichlor-pyrimidin [D.T. Hurst, Heterocycl. 22, 79 (1984)], 0,35 ml (3,1 mmol) 2-Fluorbenzylamin und 0,46 ml (3,4 mmol) Triäthylamin in 8 ml 1-Butanol wird 24 Stunden unter Rückfluss erhitzt. Anschliessend wird das Reaktionsgemisch auf 0° gekühlt. Die entstandene beigefarbene Suspension wird abgesaugt und der Filterkuchen mit Cyclohexan gewaschen und 30 Minuten bei Raumtemperatur unter Rühren in Wasser suspendiert. Die Suspension wird erneut abgesaugt und der Filterkuchen im Hochvakuum bei 100° über Tetraphosphordekaoxid getrocknet. Man erhält so das 5-Amino-2-chlor-4-[N-(2-fluorbenzyl)amino]-pyrimidin in Form von beigefarbenen Kristallen, die einen Schmelzbereich von 224 bis 225° aufweisen.

Beispiel 8:

In analoger Weise wie in Beispiel 2 beschrieben kann man durch Umsetzung von 2-Chlor-9-(2-fluorbenzyl)-9H-purin (Beispiel 7) mit N-Methylamin auch das 9-(2-Fluorbenzyl)-2-(N-methylamino)-9H-purin, das einen Schmelzbereich von 184 bis 185° (Methanol/Diäthyläther) aufweist, erhalten, wobei das Reaktionsgemisch 24 Stunden auf 80 bis 85° erwärmt wird.

Beispiel 9:

In analoger Weise wie in Beispiel 2 beschrieben kann man durch Umsetzung von 2-Chlor-9-(2-fluorbenzyl)-9H-purin (Beispiel 7) mit N,N-Dimethylamin auch das 2-(N,N-Dimethylamino)-9-(2-fluorbenzyl)-9H-purin, das einen Schmelzbereich von 112 bis 113° (Methanol/Diäthyläther) aufweist, erhalten, wobei das Reaktionsgemisch 24 Stunden auf 80 bis 85° erwärmt wird.

Beispiel 10:

In analoger Weise wie in Beispiel 2 beschrieben kann man durch Umsetzung von 2-Chlor-9-(2-fluorbenzyl)-6-(N-methylamino)-9H-purin (Beispiel 6) mit Ammoniak auch das 2-Amino-9-(2-fluorbenzyl)-6-(N-methylamino)-9H-purin erhalten, das einen Schmelzbereich von 207 bis 208° aufweist.

Beispiel 11:

In analoger Weise wie in Beispiel 10 beschrieben kann man durch Umsetzung mit N-Methylamin anstelle von Ammoniak auch das 2,6-Bis(N-methylamino)-9-(2-fluorbenzyl)-9H-purin erhalten, das einen Schmelzbereich von 170 bis 171° aufweist.

Beispiel 12:

In analoger Weise wie in Beispiel 10 beschrieben kann man durch Umsetzung mit N,N-Dimethylamin anstelle von Ammoniak auch das 2-(N,N-Dimethylamino)-9-(2-fluorbenzyl)-6-(N-methylamino)-9H-purin erhalten, das einen Schmelzbereich von 180 bis 181° aufweist.

Beispiel 13:

In analoger Weise wie in den Beispielen 1 bis 12 beschrieben kann man auch das 2,6-Diamino-9-(2-fluorbenzyl)-9H-purin erhalten.

Beispiel 14:

In analoger Weise wie in den Beispielen 1 bis 12 beschrieben kann man auch das 6-Amino-9-(2-fluorbenzyl)-2-(N-methylamino)-9H-purin erhalten.

Beispiel 15:

In analoger Weise wie in den Beispielen 1 bis 12 beschrieben kann man auch das 6-Amino-2-(N,N-dimethylamino)-9-(2-fluorbenzyl)-9H-purin erhalten.

Beispiel 16:

In analoger Weise wie in den Beispielen 1 bis 12 beschrieben kann man auch das 2-(N-Aethylamino)-6-amino-9-(2-fluorbenzyl)-9H-purin erhalten.

Beispiel 17:

In analoger Weise wie in Beispiel 1 beschrieben kann man durch Umsetzung von 2-Chlor-6-(N-methylamino)-9H-purin (Beispiel 6) mit 2,6-Difluorbenzylbromid das 2-Chlor-9-(2,6-difluorbenzyl)-6-(N-methylamino)-9H-purin erhalten, das bei 194 bis 195° (Methanol/Diäthyläther) schmilzt.

Beispiel 18:

In analoger Weise wie in Beispiel 17 beschrieben kann man durch Umsetzung von 2-Chlor-6-(N-methylamino)-9H-purin (Beispiel 6) mit 2-Chlorbenzylchlorid das 2-Chlor-9-(2-chlorbenzyl)-6-(N-methylamino)-9H-purin erhalten, das bei 203 bis 204° (N,N-Dimethylformamid/Diäthyläther) schmilzt.

Beispiel 19:

In analoger Weise wie in Beispiel 2 beschrieben kann man durch Umsetzung von 2-Chlor-9-(2,6-difluorbenzyl)-6-(N-methylamino)-9H-purin (Beispiel 17) mit N,N-Dimethylamin das 9-(2,6-Difluorbenzyl)-2-(N,N-dimethylamino)-6-(N-methylamino)-9H-purin, das einen Schmelzbereich von 196 bis 199° (Methanol) aufweist, erhalten, wobei das Reaktionsgemisch 24 Stunden auf 140° erwärmt wird.

Beispiel 20:

In analoger Weise wie in Beispiel 2 beschrieben kann man durch Umsetzung von 2-Chlor-9-(2-chlorbenzyl)-6-(N-methylamino)-9H-purin (Beispiel 18) mit N-Methylamin das 2,6-Bis(N-methylamino)-9-(2-chlorbenzyl)-9H-purin, das einen Schmelzbereich von 151 bis 152° aufweist, erhalten, wobei das Reaktionsgemisch 20 Stunden auf 120° erwärmt wird.

Beispiel 21:

In analoger Weise wie in den Beispielen 1 bis 20 beschrieben kann man unter Verwendung entsprechender 2-Chlorbenzyl-haltiger Ausgangsstoffe auch die folgenden Verbindungen erhalten:
2-Chlor-9-(2-chlorbenzyl)-6-(N,N-dimethylamino)-9H-purin; 9-(2-Chlorbenzyl)-6-(N,N-dimethylamino)-2-(N-methylamino)-9H-purin; 2-Amino-9-(2-chlorbenzyl)-6-(N,N-dimethylamino)-9H-purin; 2-(N-Aethylamino)-9-(2-chlorbenzyl)-6-(N,N-dimethylamino)-9H-purin; 9-(2-Chlorbenzyl)-2-(N-methylamino)-9H-purin; 9-(2-Chlorbenzyl)-2-(N,N-dimethylamino)-9H-purin; 2,6-Bis(N,N-dimethylamino)-9-(2-chlorbenzyl)-9H-purin; 2-Amino-9-(2-chlorbenzyl)-6-(N-methylamino)-9H-purin; 9-(2-Chlorbenzyl)-2-(N,N-dimethylamino)-6-(N-methylamino)-9H-purin; 2,6-Diamino-9-(2-chlorbenzyl)-9H-purin; 6-Amino-9-(2-chlorbenzyl)-2-(N-methylamino)-9H-purin; 6-Amino-9-(2-chlorbenzyl)-2-(N,N-dimethylamino)-9H-purin; 2-(N-Aethylamino)-6-amino-9-(2-chlorbenzyl)-9H-purin und 2-Chlor-9-(2-chlorbenzyl)-9H-purin.

Beispiel 22:

In analoger Weise wie in den Beispielen 1 bis 20 beschrieben kann man unter Verwendung entsprechender 2,6-Difluorbenzyl-haltiger Ausgangsstoffe auch die folgenden Verbindungen erhalten:
2-Chlor-9-(2,6-difluorbenzyl)-6-(N,N-dimethylamino)-9H-purin; 9-(2,6-Difluorbenzyl)-6-(N,N-dimethylamino)-2-(N-methylamino)-9H-purin; 2-Amino-9-(2,6-difluorbenzyl)-6-(N,N-dimethylamino)-9H-purin; 2-(N-Aethylamino)-9-(2,6-difluorbenzyl)-6-(N,N-dimethylamino)-9H-purin; 9-(2,6-Difluorbenzyl)-2-(N-methylamino)-9H-purin; 9-(2,6-Difluorbenzyl)-2-(N,N-dimethylamino)-9H-purin; 2,6-Bis(N,N-dimethylamino)-9-(2,6-difluorbenzyl)-9H-purin; 2-Amino-9-(2,6-difluorbenzyl)-6-(N-methylamino)-9H-purin; 2,6-Bis(N-methylamino)-9-(2,6-difluorbenzyl)-9H-purin; 2,6-Diamino-9-(2,6-difluorbenzyl)-9H-purin; 6-Amino-9-(2,6-difluorbenzyl)-2-(N-methylamino)-9H-purin; 6-Amino-9-(2,6-difluorbenzyl)-2-(N,N-dimethylamino)-9H-purin, 2-(N-Aethylamino)-6-amino-9-(2,6-difluorbenzyl)-9H-purin und 2-Chlor-9-(2,6-difluorbenzyl)-9H-purin.

Beispiel 23:

In analoger Weise wie in den Beispielen 1 bis 22 beschrieben kann man auch erhalten:
6-(N-Acetylamino)-9-(2-fluorbenzyl)-2-(N-methylamino)-9H-purin; 6-(N-Acetylamino)-9-(2-chlorbenzyl)-2-(N-methylamino)-9H-purin und 6-(N-Acetylamino)-9-(2 6-difluorbenzyl)-2-(N-methylamino)-9H-purin.

Beispiel 24:

Tabletten, enthaltend je 50 mg des Wirkstoffs, z.B. das 6-(N,N-Dimethylamino)-9-(2-fluorbenzyl)-2-(N-methylamino)-9H-purin oder ein pharmazeutisch verwendbares Salz davon, können wie folgt hergestellt werden:

| Zusammensetzung (für 10000 Tabletten): | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdispers) | 20.0 g |
| Aethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 25:

Lacktabletten, enthaltend je 200 mg des Wirkstoffs, z.B. das 6-(N,N-Dimethylamino)-9-(2-fluorbenzyl)-2-(N-methylamino)-9H-purin oder ein pharmazeutisch verwendbares Salz davon, können wie folgt hergestellt werden:

| Zusammensetzung (für 500 Tabletten): | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropylmethylcellulose | 1.18 g |
| Schellack | 0.32 g |
| Wasser | q.s. |
| Dichlormethan | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 560 mg) verpresst und diese mit einer Lösung der Hydroxypropylmethylcellulose und des Schellacks in Dichlormethan lackiert; Endgewicht der Lacktablette: 563 mg.

Beispiel 26:

In analoger Weise wie in den Beispielen 24 und 25 beschrieben können auch pharmazeutische Präparate, enthaltend eine andere Verbindung der Formel I oder ein pharmazeutisch verwendbares Salz davon, beispielsweise gemäss den Beispielen 1 bis 23, hergestellt werden.

**Ansprüche**

1. Eine Verbindung der Formel

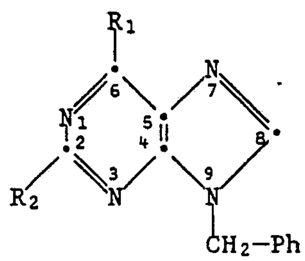

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, mit der Massgabe, dass $R_2$ von Halogen verschieden ist, wenn Ph 2-Fluorphenyl oder 2,5- oder 2,6-Difluorphenyl ist und $R_1$ einen Rest der Formel $-N(R_{11})(R_{12})$ (Ia), worin entweder $R_{11}$ Wasserstoff, Methyl oder Aethyl ist und $R_{12}$ Wasserstoff, Methyl, Hydroxymethyl, Aethyl, n-Propyl, Isopropyl, Cyclopropyl, Cyclopentyl oder Cyclopropylmethyl ist oder worin $R_{11}$ Wasserstoff ist und $R_{12}$ Methoxymethyl ist, darstellt, und mit der weiteren Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, sowie eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon.

2. Eine Verbindung gemäss Anspruch 1 der Formel I, worin, unter Berücksichtigung der in Anspruch 1 erwähnten Massgaben, Ph einen durch Halogen substituierten Phenylrest bedeutet, $R_1$ Wasserstoff, Amino, N-Mono- oder N,N-Diniederalkylamino, N-(Niederalkoxyniederalkyl)amino, N-(Hydroxyniederalkyl)amino, N-(Hydroxyniederalkyl)-N-niederalkyl-amino, N-Mono-oder N,N-Dicycloalkylamino, N-Cycloalkyl-N-niederalkyl-amino, N-Mono- oder N,N-Di(cycloalkylniederalkyl)amino, N-(Cycloalkylniederalkyl)-N-niederalkyl-amino, N-Niederalkanoylamino oder N-Niederalkanoyl-N-niederalkyl-amino darstellt und $R_2$ für Halogen, Niederalkoxy, Niederalkyl, Amino, N-Mono- oder N,N-Diniederalkylamino, N-(Niederalkoxyniederalkyl)amino, N-(Hydroxyniederalkyl)amino, N-(Hydroxyniederalkyl)-N-niederalkylamino, N-Mono- oder N,N-Dicycloalkylamino, N-Cycloalkyl-N-niederalkylamino, N-Mono- oder N,N-Di(cycloalkylniederalkyl)amino, N-(Cycloalkylniederalkyl)-N-niederalkyl-amino, N-Niederalkanoylamino oder N-Niederalkanoyl-N-niederalkyl-amino steht, sowie eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon.

3. Eine Verbindung gemäss Anspruch 1 der Formel I, worin, unter Berücksichtigung der in Anspruch 1 erwähnten Massgaben, Ph 2-, 3- oder 4-Halogenphenyl, 2,3-, 2,5- oder 2,6-Dihalogenphenyl oder 2,3,6- oder 2,5,6-Trihalogenphenyl bedeutet, wobei Halogen jeweils Halogen mit einer Atomnummer bis und mit 35 sein kann, $R_1$ Wasserstoff, Amino, N-$C_1$-$C_4$-Alkylamino, N,N-Di-$C_1$-$C_4$-alkylamino, N-$C_3$-$C_6$-Cycloalkylamino oder N-Niederalkanoylamino darstellt und $R_2$ für Halogen mit einer Atomnummer bis und mit 35, $C_1$-$C_4$-Alkoxy, $C_1$-$C_4$-Alkyl, Amino, N-$C_1$-$C_4$-Alkylamino, N,N-Di-$C_1$-$C_4$-alkylamino, N-$C_3$-$C_6$-Cycloalkylamino oder N-Niederalkanoylamino steht, sowie eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon.

4. Eine Verbindung gemäss Anspruch 1 der Formel I, worin, unter Berücksichtigung der in Anspruch 1 erwähnten Massgaben, Ph 2-Halogenphenyl oder 2,6-Dihalogenphenyl bedeutet, wobei Halogen jeweils Halogen mit einer Atomnummer bis und mit 35 sein kann, $R_1$ Wasserstoff, Amino, N-$C_1$-$C_4$-Alkylamino, N,N-Di-$C_1$-$C_4$-alkylamino oder N-Niederalkanoylamino darstellt und $R_2$ für Halogen mit einer Atomnummer bis und mit 35, Amino, N-$C_1$-$C_4$-Alkylamino oder N,N-Di-$C_1$-$C_4$-alkylamino steht, sowie eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon.

5. Eine Verbindung gemäss Anspruch 1 der Formel I, worin, unter Berücksichtigung der in Anspruch 1 erwähnten Massgaben, Ph 2-Fluorphenyl, 2-Chlorphenyl oder 2,6-Difluorphenyl bedeutet, $R_1$ Wasserstoff, Amino, N-$C_1$-$C_4$-Alkylamino oder N,N-Di-$C_1$-$C_4$-alkylamino darstellt und $R_2$ für Halogen mit einer Atomnummer bis und mit 35, Amino, N-$C_1$-$C_4$-Alkylamino oder N,N-Di-$C_1$-$C_4$-alkylamino steht, sowie eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon.

6. Eine Verbindung gemäss Anspruch 1 der Formel I, worin, unter Berücksichtigung der in Anspruch 1 erwähnten Massgaben, Ph 2-Fluorphenyl oder 2,6-Difluorphenyl bedeutet, $R_1$ N-$C_1$-$C_4$-Alkylamino oder N,N-Di-$C_1$-$C_4$-alkylamino darstellt und $R_2$ für Halogen mit einer Atomnummer bis und mit 35, Amino, N-$C_1$-$C_4$-Alkylamino oder N,N-Di-$C_1$-$C_4$-alkylamino steht, sowie eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon.

7. Eine Verbindung gemäss Anspruch 1 der Formel I, worin, unter Berücksichtigung der in Anspruch 1 erwähnten Massgaben, Ph 2-Fluorphenyl bedeutet, $R_1$ N,N-Di-$C_1$-$C_4$-alkylamino darstellt und $R_2$ für Halogen mit einer Atomnummer bis und mit 35, Amino oder N-$C_1$-$C_4$-Alkylamino steht, sowie die neue Verbindung der Formel I, worin Ph 2-Fluorphenyl ist, $R_1$ N,N-Dimethylamino ist und $R_2$ für Chlor steht, oder jeweils ein Salz davon.

8. Eine Verbindung gemäss Anspruch 1 der Formel I, worin Ph 2-Fluorphenyl bedeutet, $R_1$ N,N-Di-$C_1$-$C_4$-alkylamino darstellt und $R_2$ für N-$C_1$-$C_4$-Alkylamino steht, oder ein Salz davon.

9. 6-(N,N-Dimethylamino)-9-(2-fluorbenzyl)-2-(N-methylamino)-9H-purin oder ein Salz davon.

10. 2-Chlor-9-(2-fluorbenzyl)-6-(N-methylamino)-9H-purin oder ein Salz davon.

11. 2-Amino-9-(2-fluorbenzyl)-6-(N-methylamino)-9H-purin oder ein Salz davon.

12. 2,6-Bis(N-methylamino)-9-(2-fluorbenzyl)-9H-purin oder ein Salz davon.

13. 9-(2,6-Difluorbenzyl)-2-(N,N-dimethylamino)-6-(N-methylamino)-9H-purin oder ein Salz davon.

14. 2-Chlor-6-(N,N-dimethylamino)-9-(2-fluorbenzyl)-9H-purin, 2-Amino-6-(N,N-dimethylamino)-9-(2-fluorbenzyl)-9H-purin, 2-(N-Aethylamino)-6-(N,N-dimethylamino)-9-(2-fluorbenzyl)-9H-purin, 2,6-Bis(N,N-dimethylamino)-9-(2-fluorbenzyl)-9H-purin, 2-Chlor-9-(2-fluorbenzyl)-9H-purin, 9-(2-Fluorbenzyl)-2-(N-methylamino)-9H-purin, 2-(N,N-Dimethylamino)-9-(2-fluorbenzyl)-9H-purin, 2-(N,N-Dimethylamino)-9-(2-fluorbenzyl)-6-(N-methylamino)-9H-purin, 2,6-Diamino-9-(2-fluorbenzyl)-9H-purin, 2-Chlor-9-(2,6-difluorbenzyl)-6-(N-methylamino)-9H-purin, 6-Amino-9-(2-fluorbenzyl)-2-(N-methylamino)-9H-purin, 2-Chlor-9-(2-chlorbenzyl)-6-(N-methylamino)-9H-purin, 6-Amino-2-(N,N-dimethylamino)-9-(2-fluorbenzyl)-9H-purin, 2-(N-Aethylamino)-6-amino-9-(2-fluorbenzyl)-9H-purin oder 2,6-Bis(N-methylamino)-9-(2-chlorbenzyl)-9H-purin oder jeweils ein Salz davon.

15. Eine Verbindung der Formel

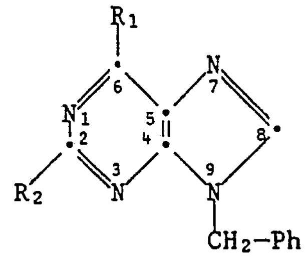

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, oder ein pharmazeutisch verwendbares Salz davon zur Anwendung in einem Verfahren zur therapeutischen und/oder prophylaktischen Behandlung des menschlichen oder tierischen Körpers.

16. Eine Verbindung gemäss Anspruch 15, mit der Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, oder ein pharmazeutisch verwendbares Salz davon zur Anwendung in einem Verfahren zur therapeutischen und/oder prophylaktischen Behandlung des menschlichen oder tierischen Körpers.

17. Eine Verbindung gemäss einem der Ansprüche 1 bis 14 oder ein pharmazeutisch verwendbares Salz davon zur Anwendung in einem Verfahren zur therapeutischen und/oder prophylaktischen Behandlung des menschlichen oder tierischen Körpers.

18. Eine Verbindung gemäss einem der Ansprüche 1 bis 5 und 7 bis 14 oder ein pharmazeutisch verwendbares Salz davon zur Anwendung in einem Verfahren zur therapeutischen und/oder prophylaktischen Behandlung des menschlichen oder tierischen Körpers.

19. Eine Verbindung gemäss einem der Ansprüche 1 bis 18 oder ein pharmazeutisch verwendbares Salz davon zur Anwendung als Antikonvulsivum.

20. Eine Verbindung gemäss einem der Ansprüche 1 bis 5, 7 bis 14 und 18 oder ein pharmazeutisch verwendbares Salz davon zur Anwendung als Antikonvulsivum.

21. Ein pharmazeutisches Präparat, als Wirkstoff enthaltend eine Verbindung gemäss einem der Ansprüche 1 bis 20 oder ein pharmazeutisch verwendbares Salz davon gegebenenfalls neben üblichen pharmazeutischen Hilfsstoffen.

22. Ein pharmazeutisches Präparat, als Wirkstoff enthaltend eine Verbindung gemäss einem der Ansprüche 1 bis 5, 7 bis 14, 18 und 20 oder ein pharmazeutisch verwendbares Salz davon gegebenenfalls neben üblichen pharmazeutischen Hilfsstoffen.

23. Ein antikonvulsiv wirksames pharmazeutisches Präparat gemäss Anspruch 21 oder 22, dadurch gekennzeichnet, dass man einen antikonvulsiv wirksamen Wirkstoff wählt.

24. Ein antikonvulsiv wirksames pharmazeutisches Präparat gemäss Anspruch 22, dadurch gekennzeichnet, dass man einen antikonvulsiv wirksamen Wirkstoff wählt.

25. Verfahren zur Herstellung einer Verbindung der Formel

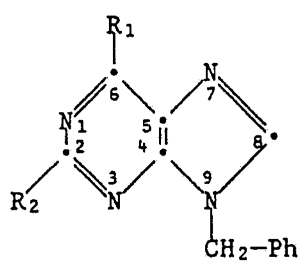

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, mit der Massgabe, dass $R_2$ von Halogen verschieden ist, wenn Ph 2-Fluorphenyl oder 2,5- oder 2,6-Difluorphenyl ist und $R_1$ einen Rest der Formel $-N(R_{11})(R_{12})$ (Ia), worin entweder $R_{11}$ Wasserstoff, Methyl oder Aethyl ist und $R_{12}$

Wasserstoff, Methyl, Hydroxymethyl, Aethyl, n-Propyl, Isopropyl, Cyclopropyl, Cyclopentyl oder Cyclopropylmethyl ist oder worin $R_{11}$ Wasserstoff ist und $R_{12}$ Methoxymethyl ist, darstellt, und mit der weiteren Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, oder einer neuen Verbin dung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils eines Salzes davon, dadurch gekennzeichnet, dass man

a) in einer Verbindung der Formel

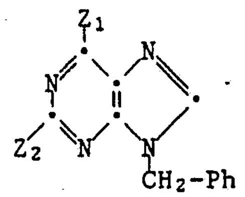

(II),

worin $Z_1$ eine nucleofuge Abgangsgruppe $X_1$ ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abgangsgruppe $X_2$ steht, oder einem Tautomeren und/oder Salz davon $Z_1$ in $R_1$ und/oder $Z_2$ in $R_2$ überführt oder

b) aus einer Verbindung der Formel

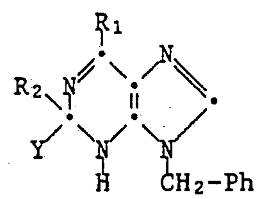

(III),

worin Y Hydroxy, Mercapto oder gegebenenfalls aliphatisch substituiertes Amino bedeutet, oder einem Tautomeren und/oder Salz davon die Verbindung Y-H eliminiert oder

c) zur Herstellung einer Verbindung 1, worin $R_2$ für Amino steht, oder eines Salzes davon eine Verbindung der Formel

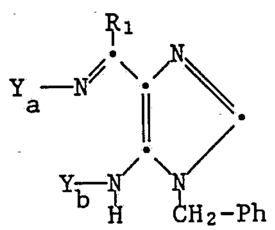

(IV),

worin einer der Reste $Y_a$ und $Y_b$ Cyano und der andere Wasserstoff bedeutet, oder ein Tautomeres und/oder Salz davon cyclisiert oder

d) eine Verbindung der Formel

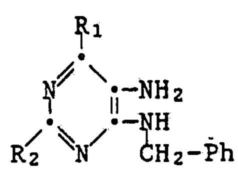

(V)

oder ein Salz davon mit Ameisensäure oder einem reaktionsfähigen Derivat davon umsetzt oder

e) eine Verbindung der Formel

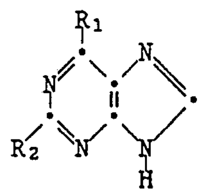

(VI)

oder ein Salz davon mit einer Verbindung der Formel $X_1$-$CH_2$-Ph (VII), worin $X_1$ eine nucleofuge Abgangsgruppe darstellt, umsetzt oder

f) zur Herstellung einer Verbindung I, worin mindestens einer der Reste $R_1$ und $R_2$ für Amino steht, oder eines Salzes davon in einer Verbindung der Formel

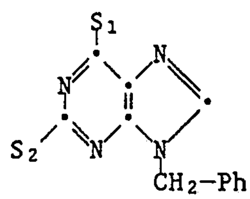

(VIII),

worin $S_1$ eine Aminoschutzgruppe bedeutet und $S_2$ eine Aminoschutzgruppe oder ein Rest $R_2$ ist oder worin $S_1$ einen Rest $R_1$ darstellt und $S_2$ eine Aminoschutzgruppe bedeutet, oder einem Salz davon $S_1$ in $R_1$ und/oder $S_2$ in $R_2$ überführt und gewünschtenfalls jeweils ein gegebenenfalls verfahrensgemäss erhältliches Isomerengemisch in die Komponenten auftrennt und das Isomere der Formel I isoliert, eine verfahrensgemäss oder auf andere Weise erhältliche Verbindung I in eine andere Verbindung I überführt, eine verfahrensgemäss erhältliche freie Verbindung I in ein Salz überführt oder ein verfahrensgemäss erhältliches Salz einer Verbindung I in die freie Verbindung I oder in ein anderes Salz der Verbindung I umwandelt und/oder ein gegebenenfalls verfahrensgemäss erhältliches Stereoisomerengemisch in die Stereoisomeren auftrennt und das gewünschte Stereoisomere isoliert.

26. Verfahren zur Herstellung eines pharmazeutischen Präparates gemäss Anspruch 21, dadurch gekennzeichnet, dass man den Wirkstoff, gegebenenfalls unter Beimischung von üblichen pharmazeutischen Hilfsstoffen, zu einem pharmazeutischen Präparat verarbeitet.

27. Verfahren gemäss Anspruch 26 zur Herstellung eines antikonvulsiv wirksamen pharmazeutischen Präparates gemäss Anspruch 23, dadurch gekennzeichnet, dass man einen antikonvulsiv wirksamen Wirkstoff wählt.

28. Verwendung einer Verbindung gemäss einem der Ansprüche 1 bis 20 oder eines pharmazeutisch verwendbaren Salzes davon zur Herstellung eines pharmazeutischen Präparats.

29. Verwendung einer Verbindung gemäss Anspruch 28 zur Herstellung eines Antikonvulsivums.

30. Verwendung einer Verbindung gemäss einem der Ansprüche 1 bis 20 oder eines pharmazeutisch verwendbaren Salzes davon zur Herstellung eines pharmazeutischen Präparats auf nicht-chemischem Wege.

Patentansprüche für folgende Vertragsstaaten: ES, GR

1. Verfahren zur Herstellung einer Verbindung der Formel

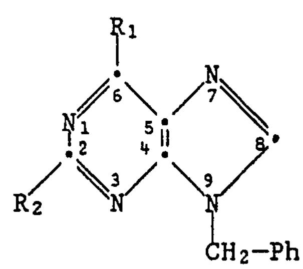

(I),

worin Ph einen durch Halogen substituierten Phenylrest bedeutet, $R_1$ Wasserstoff oder eine freie oder

aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, mit der Massgabe, dass $R_2$ von Halogen verschieden ist, wenn Ph 2-Fluorphenyl oder 2,5- oder 2,6-Difluorphenyl ist und $R_1$ einen Rest der Formel $-N(R_{11})(R_{12})$ (Ia), worin entweder $R_{11}$ Wasserstoff, Methyl oder Aethyl ist und $R_{12}$ Wasserstoff, Methyl, Hydroxymethyl, Aethyl, n-Propyl, Isopropyl, Cyclopropyl, Cyclopentyl oder Cyclopropylmethyl ist oder worin $R_{11}$ Wasserstoff ist und $R_{12}$ Methoxymethyl ist, darstellt, und mit der weiteren Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, oder einer neuen Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils eines Salzes davon, dadurch gekennzeichnet, dass man

a) in einer Verbindung der Formel

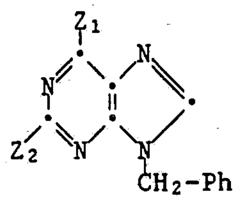

(II),

worin $Z_1$ eine nucleofuge Abgangsgruppe $X_1$ ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abgangsgruppe $X_2$ steht, oder einem Tautomeren und/oder Salz davon $Z_1$ in $R_1$ und/oder $Z_2$ in $R_2$ überführt oder

b) aus einer Verbindung der Formel

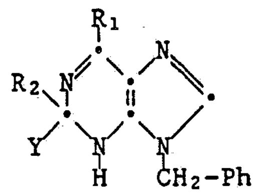

(III),

worin Y Hydroxy, Mercapto oder gegebenenfalls aliphatisch substituiertes Amino bedeutet, oder einem Tautomeren und/oder Salz davon die Verbindung Y-H eliminiert oder

c) zur Herstellung einer Verbindung I, worin $R_2$ für Amino steht, oder eines Salzes davon eine Verbindung der Formel

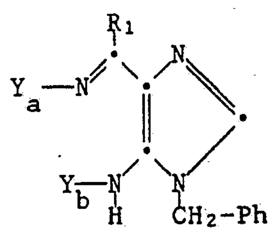

(IV),

worin einer der Reste $Y_a$ und $Y_b$ Cyano und der andere Wasserstoff bedeutet, oder ein Tautomeres und/oder Salz davon cyclisiert oder

d) eine Verbindung der Formel

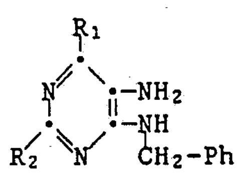

(V)

oder ein Salz davon mit Ameisensäure oder einem reaktionsfähigen Derivat davon umsetzt oder

e) eine Verbindung der Formel

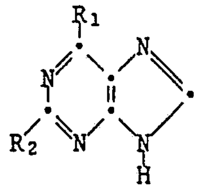

(VI)

oder ein Salz davon mit einer Verbindung der Formel $X_1$-$CH_2$-Ph (VII), worin $X_1$ eine nucleofuge Abgangsgruppe darstellt, umsetzt oder

f) zur Herstellung einer Verbindung I, worin mindestens einer der Reste $R_1$ und $R_2$ für Amino steht, oder eines Salzes davon in einer Verbindung der Formel

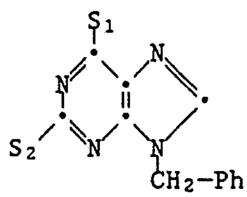

(VIII),

worin $S_1$ eine Aminoschutzgruppe bedeutet und $S_2$ eine Aminoschutzgruppe oder ein Rest $R_2$ ist oder worin $S_1$ einen Rest $R_1$ darstellt und $S_2$ eine Aminoschutzgruppe bedeutet, oder einem Salz davon $S_1$ in $R_1$ und/oder $S_2$ in $R_2$ überführt und gewünschtenfalls jeweils ein gegebenenfalls verfahrensgemäss erhältliches Isomerengemisch in die Komponenten auftrennt und das Isomere der Formel I isoliert, eine verfahrensgemäss oder auf andere Weise erhältliche Verbindung I in eine andere Verbindung I überführt, eine verfahrensgemäss erhältliche freie Verbindung I in ein Salz überführt oder ein verfahrensgemäss erhältliches Salz einer Verbindung I in die freie Verbindung I oder in ein anderes Salz der Verbindung I umwandelt und/oder ein gegebenenfalls verfahrensgemäss erhältliches Stereoisomerengemisch in die Stereoisomeren auftrennt und das gewünschte Stereoisomere isoliert.

2. Verfahren gemäss Anspruch 1, dadurch gekennzeichnet, dass man eine Verbindung der Formel I, worin, unter Berücksichtigung der in Anspruch 1 erwähnten Massgaben, Ph einen durch Halogen substituierten Phenylrest bedeutet, $R_1$ Wasserstoff, Amino, N-Mono- oder N,N-Diniederalkylamino, N-(Niederalkoxyniederalkyl)amino, N-(Hydroxyniederalkyl)amino, N-(Hydroxyniederalkyl)-N-niederalkyl-amino, N-Mono-oder N,N-Dicycloalkylamino, N-Cycloalkyl-N-niederalkyl-amino, N-Mono-oder N,N-Di-(cycloalkylniederalkyl)amino, N-(Cycloalkylniederalkyl)-N-niederalkyl-amino, N-Niederalkanoylamino oder N-Niederalkanoyl-N-niederalkyl-amino darstellt und $R_2$ für Halogen, Niederalkoxy, Niederalkyl, Amino, N-Mono- oder N,N-Diniederalkylamino, N-(Niederalkoxyniederalkyl)amino, N-(Hydroxyniederalkyl)amino, N-(Hydroxyniederalkyl)-N-niederalkyl-amino, N-Mono- oder N,N-Dicycloalkylamino, N-Cycloalkyl-N-niederalkyl-amino, N-Mono- oder N,N-Di(cycloalkylniederalkyl)amino, N-(Cycloalkylniederalkyl)-N-niederalkyl-amino, N-Niederalkanoylamino oder N-Niederalkanoyl-N-niederalkylamino steht, oder eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon herstellt.

3. Verfahren gemäss Anspruch 1, dadurch gekennzeichnet, dass man eine Verbindung der Formel I, worin, unter Berücksichtigung der in Anspruch 1 erwähnten Massgaben, Ph 2-, 3- oder 4-Halogenphenyl, 2,3-, 2,5- oder 2,6-Dihalogenphenyl oder 2,3,6- oder 2,5,6-Trihalogenphenyl bedeutet, wobei Halogen jeweils Halogen mit einer Atomnummer bis und mit 35 sein kann, $R_1$ Wasserstoff, Amino, N-$C_1$-$C_4$-Alkylamino, N,N-Di-$C_1$-$C_4$-alkylamino, N-$C_3$-$C_6$-Cycloalkylamino oder N-Niederalkanoylamino darstellt und $R_2$ für Halogen mit einer Atomnummer bis und mit 35, $C_1$-$C_4$-Alkoxy, $C_1$-$C_4$-Alkyl, Amino, N-$C_1$-$C_4$-Alkylamino, N,N-Di-$C_1$-$C_4$-alkylamino, N-$C_3$-$C_6$-Cycloalkylamino oder N-Niederalkanoylamino steht, oder eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon herstellt.

4. Verfahren gemäss Anspruch 1, dadurch gekennzeichnet, dass man eine Verbindung der Formel I, worin, unter Berücksichtigung der in Anspruch 1 erwähnten Massgaben, Ph 2-Halogenphenyl oder 2,6-Dihalogenphenyl bedeutet, wobei Halogen jeweils Halogen mit einer Atomnummer bis und mit 35 sein kann,

$R_1$ Wasserstoff, Amino, N-$C_1$-$C_4$-Alkylamino, N,N-Di-$C_1$-$C_4$-alkylamino oder N-Niederalkanoylamino darstellt und $R_2$ für Halogen mit einer Atomnummer bis und mit 35, Amino, N-$C_1$-$C_4$-Alkylamino oder N,N-Di-$C_1$-$C_4$-alkylamino steht, oder eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon herstellt.

5. Verfahren gemäss Anspruch 1, dadurch gekennzeichnet, dass man eine Verbindung der Formel I, worin, unter Berücksichtigung der in Anspruch 1 erwähnten Massgaben, Ph 2-Fluorphenyl, 2-Chlorphenyl oder 2,6-Difluorphenyl bedeutet, $R_1$ Wasserstoff, Amino, N-$C_1$-$C_4$-Alkylamino oder N,N-Di-$C_1$-$C_4$-alkylamino darstellt und $R_2$ für Halogen mit einer Atomnummer bis und mit 35, Amino, N-$C_1$-$C_4$-Alkylamino oder N,N-Di-$C_1$-$C_4$-alkylamino steht, oder eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon herstellt.

6. Verfahren gemäss Anspruch 1, dadurch gekennzeichnet, dass man eine Verbindung der Formel I, worin, unter Berücksichtigung der in Anspruch 1 erwähnten Massgaben, Ph 2-Fluorphenyl oder 2,6-Difluorphenyl bedeutet, $R_1$ N-$C_1$-$C_4$-Alkylamino oder N,N-Di-$C_1$-$C_4$-alkylamino darstellt und $R_2$ für Halogen mit einer Atomnummer bis und mit 35, Amino, N-$C_1$-$C_4$-Alkylamino oder N,N-Di-$C_1$-$C_4$-alkylamino steht, oder eine neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, oder jeweils ein Salz davon herstellt.

7. Verfahren gemäss Anspruch 1, dadurch gekennzeichnet, dass man eine Verbindung der Formel I, worin, unter Berücksichtigung der in Anspruch 1 erwähnten Massgaben, Ph 2-Fluorphenyl bedeutet, $R_1$ N,N-Di-$C_1$-$C_4$-alkylamino darstellt und $R_2$ für Halogen mit einer Atomnummer bis und mit 35, Amino oder N-$C_1$-$C_4$-Alkylamino steht, oder die neue Verbindung der Formel I, worin Ph 2-Fluorphenyl ist, $R_1$ N,N-Dimethylamino ist und $R_2$ für Chlor steht, oder jeweils ein Salz davon herstellt.

8. Verfahren gemäss Anspruch 1, dadurch gekennzeichnet, dass man eine Verbindung der Formel I, worin Ph 2-Fluorphenyl bedeutet, $R_1$ N,N-Di-$C_1$-$C_4$-alkylamino darstellt und $R_2$ für N-$C_1$-$C_4$-Alkylamino steht, oder ein Salz davon herstellt.

9. Verfahren gemäss Anspruch 1, dadurch gekennzeichnet, dass man 6-(N,N-Dimethylamino)-9-(2-fluorbenzyl)-2-(N-methylamino)-9H-purin oder ein Salz davon herstellt.

10. Verfahren gemäss Anspruch 1, dadurch gekennzeichnet, dass man 2-Chlor-9-(2-fluorbenzyl)-6-(N-methylamino)-9H-purin oder ein Salz davon herstellt.

11. Verfahren gemäss Anspruch 1, dadurch gekennzeichnet, dass man 2-Amino-9-(2-fluorbenzyl)-6-(N-methylamino)-9H-purin oder ein Salz davon herstellt.

12. Verfahren gemäss Anspruch 1, dadurch gekennzeichnet, dass man 2,6-Bis(N-methylamino)-9-(2-fluorbenzyl)-9H-purin oder ein Salz davon herstellt.

13. Verfahren gemäss Anspruch 1, dadurch gekennzeichnet, dass man 9-(2,6-Difluorbenzyl)-2-(N,N-dimethylamino)-6-(N-methylamino)-9H-purin oder ein Salz davon herstellt.

14. Verfahren gemäss Anspruch 1, dadurch gekennzeichnet, dass man 2-Chlor-6-(N,N-dimethylamino)-9-(2-fluorbenzyl)-9H-purin, 2-Amino-6-(N,N-dimethylamino)-9-(2-fluorbenzyl)-9H-purin, 2-(N-Aethylamino)-6-(N,N-dimethylamino)-9-(2-fluorbenzyl)-9H-purin, 2,6-Bis(N,N-dimethylamino)-9-(2-fluorbenzyl)-9H-purin, 2-Chlor-9-(2-fluorbenzyl)-9H-purin, 9-(2-Fluorbenzyl)-2-(N-methylamino)-9H-purin, 2-(N,N-Dimethylamino)-9-(2-fluorbenzyl)-9H-purin, 2-(N,N-Dimethylamino)-9-(2-fluorbenzyl)-6-(N-methylamino)-9H-purin, 2,6-Diamino-9-(2-fluorbenzyl)-9H-purin, 2-Chlor-9-(2,6-difluorbenzyl)-6-(N-methylamino)-9H-purin, 6-Amino-9-(2-fluorbenzyl)-2-(N-methylamino)-9H-purin, 2-Chlor-9-(2-chlorbenzyl)-6-(N-methylamino)-9H-purin, 6-Amino-2-(N,N-dimethylamino)-9-(2-fluor benzyl)-9H-purin, 2-(N-Aethylamino)-6-amino-9-(2-fluorbenzyl)-9H-purin oder 2,6-Bis(N-methylamino)-9-(2-chlorbenzyl)-9H-purin oder jeweils ein Salz davon herstellt.

15. Verfahren zur Herstellung eines pharmazeutischen Präparates, dadurch gekennzeichnet, dass man

a) in einer Verbindung der Formel

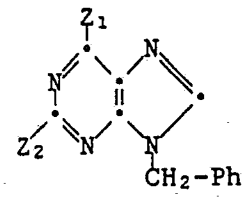

(II),

worin $Z_1$ eine nucleofuge Abgangsgruppe $X_1$ ist und $Z_2$ eine nucleofuge Abgangsgruppe $X_2$ oder einen Rest $R_2$ darstellt oder worin $Z_1$ ein Rest $R_1$ ist und $Z_2$ für eine nucleofuge Abgangsgruppe $X_2$ steht, oder

einem Tautomeren und/oder Salz davon $Z_1$ in $R_1$ und/oder $Z_2$ in $R_2$ überführt oder

b) aus einer Verbindung der Formel

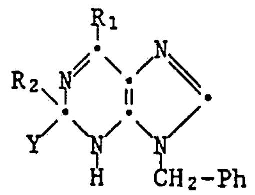

(III),

worin Y Hydroxy, Mercapto oder gegebenenfalls aliphatisch substituiertes Amino bedeutet, oder einem Tautomeren und/oder Salz davon die Verbindung Y-H eliminiert oder

c) zur Herstellung einer Verbindung I, worin $R_2$ für Amino steht, oder eines Salzes davon eine Verbindung der Formel

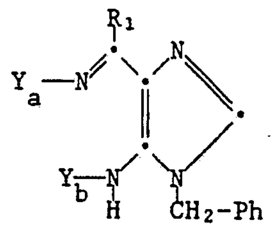

(IV),

worin einer der Reste $Y_a$ und $Y_b$ Cyano und der andere Wasserstoff bedeutet, oder ein Tautomeres und/oder Salz davon cyclisiert oder

d) eine Verbindung der Formel

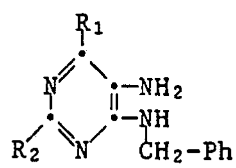

(V)

oder ein Salz davon mit Ameisensäure oder einem reaktionsfähigen Derivat davon umsetzt oder

e) eine Verbindung der Formel

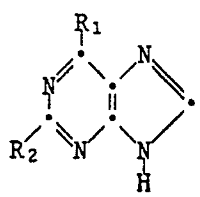

(VI)

oder ein Salz davon mit einer Verbindung der Formel $X_1$-$CH_2$-Ph (VII), worin $X_1$ eine nucleofuge Abgangsgruppe darstellt, umsetzt oder

f) zur Herstellung einer Verbindung I, worin mindestens einer der Reste $R_1$ und $R_2$ für Amino steht, oder eines Salzes davon in einer Verbindung der Formel

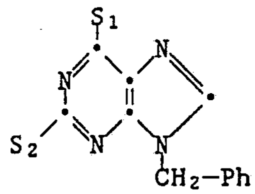

(VIII),

worin $S_1$ eine Aminoschutzgruppe bedeutet und $S_2$ eine Aminoschutzgruppe oder ein Rest $R_2$ ist oder worin $S_1$ einen Rest $R_1$ darstellt und $S_2$ eine Aminoschutzgruppe bedeutet, oder einem Salz davon $S_1$ in $R_1$ und/oder $S_2$ in $R_2$ überführt und gewünschtenfalls jeweils ein gegebenenfalls verfahrensgemäss erhältliches Isomerengemisch in die Komponenten auftrennt und das Isomere der Formel I isoliert, eine verfahrensgemäss oder auf andere Weise erhältliche Verbindung I in eine andere Verbindung I überführt, eine verfahrensgemäss erhältliche freie Verbindung I in ein Salz überführt oder ein verfahrensgemäss erhältliches Salz einer Verbindung I in die freie Verbindung I oder in ein anderes Salz der Verbindung I umwandelt und/oder ein gegebenenfalls verfahrensgemäss erhältliches Stereoisomerengemisch in die Stereoisomeren auftrennt und das gewünschte Stereoisomere isoliert und eine auf diese Weise erhaltene Verbindung der Formel

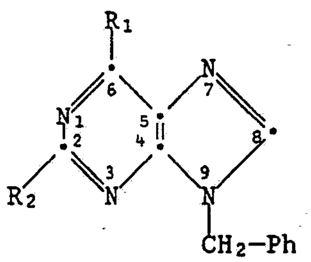

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, mit der Massgabe, dass $R_2$ von Halogen verschieden ist, wenn Ph 2-Fluorphenyl oder 2,5- oder 2,6-Difluorphenyl ist und $R_1$ einen Rest der Formel $-N(R_{11})(R_{12})$ (Ia), worin entweder $R_{11}$ Wasserstoff, Methyl oder Aethyl ist und $R_{12}$ Wasserstoff, Methyl, Hydroxymethyl, Aethyl, n-Propyl, Isopropyl, Cyclopropyl, Cyclopentyl oder Cyclopropylmethyl ist oder worin $R_{11}$ Wasserstoff ist und $R_{12}$ Methoxymethyl ist, darstellt, und mit der weiteren Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, oder eine auf diese Weise erhältliche neue Verbindung der Formel I, worin Ph 2-Fluorphenyl oder 2,6-Difluorphenyl ist, $R_1$ N-Methylamino oder N,N-Dimethylamino ist und $R_2$ für Chlor steht, jeweils in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

16. Verfahren zur Herstellung eines pharmazeutischen Präparates, dadurch gekennzeichnet, dass man eine Verbindung, erhältlich gemäss einem der Ansprüche 1 bis 14, jeweils in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

17. Verfahren zur Herstellung eines pharmazeutischen Präparates gemäss Anspruch 16, dadurch gekennzeichnet, dass man eine Verbindung, erhältlich gemäss einem der Ansprüche 1 bis 5 und 7 bis 14, jeweils in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

18. Verfahren zur Herstellung eines pharmazeutischen Präparates, dadurch gekennzeichnet, dass man eine Verbindung der Formel

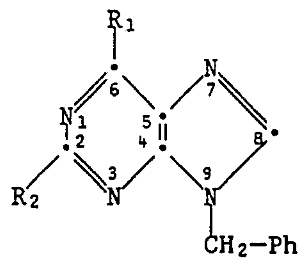

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, oder ein pharmazeutisch ver wendbares Salz davon, gegebenenfalls unter Beimischung von üblichen pharmazeutischen Hilfsstoffen, zu einem pharmazeutischen Präparat verarbeitet.

19. Verfahren zur Herstellung eines pharmazeutischen Präparates gemäss Anspruch 18, dadurch gekennzeichnet, dass man eine Verbindung der Formel

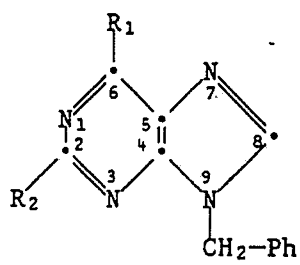

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, mit der Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, oder ein pharmazeutisch verwendbares Salz davon, gegebenenfalls unter Beimischung von üblichen pharmazeutischen Hilfsstoffen, zu einem pharmazeutischen Präparat verarbeitet.

20. Verfahren gemäss einem der Ansprüche 15 bis 19 zur Herstellung eines antikonvulsiv wirksamen pharmazeutischen Präparates, dadurch gekennzeichnet, dass man einen antikonvulsiv wirksamen Wirkstoff wählt.

21. Verfahren gemäss Anspruch 15 oder 17 zur Herstellung eines antikonvulsiv wirksamen pharmazeutischen Präparates, dadurch gekennzeichnet, dass man einen antikonvulsiv wirksamen Wirkstoff wählt.

22. Verwendung einer Verbindung, erhältlich gemäss einem der Ansprüche 1 bis 14, oder eines pharmazeutisch verwendbaren Salzes davon zur Herstellung eines pharmazeutischen Präparats.

23. Verwendung einer Verbindung der Formel

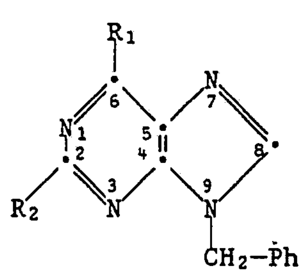

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch,

cycloaliphatisch-aliphatisch und/oder durch Acyl substituierte Aminogruppe steht, oder eines pharmazeutisch verwendbaren Salzes davon zur Herstellung eines pharmazeutischen Präparats.

24. Verwendung einer Verbindung der Formel

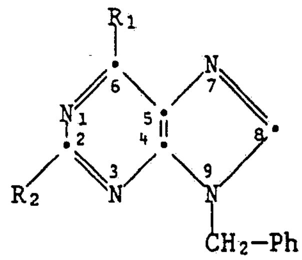

(I),

worin Ph einen durch Halogen substituierten Phenylrest bedeutet, $R_1$ Wasserstoff oder eine freie oder aliphatisch, cycloaliphatisch, cycloaliphatisch-aliphatisch und/oder durch Acyl substituierte Aminogruppe darstellt und $R_2$ für Halogen, Niederalkoxy, Niederalkyl oder eine freie oder aliphatisch, cycloaliphatisch, cycloaliphatisch-aliphatisch und/oder durch Acyl substituierte Aminogruppe steht, mit der Massgabe, dass $R_2$ von Chlor verschieden ist, wenn Ph 3-Chlorphenyl, 4-Chlorphenyl oder 3,4-Dichlorphenyl ist und $R_1$ N,N-Dimethylamino ist, oder eines pharmazeutisch verwendbaren Salzes davon zur Herstellung eines pharmazeutischen Präparats.

25. Verwendung einer Verbindung gemäss einem der Ansprüche 22 bis 24 zur Herstellung eines Antikonvulsivums.

26. Verwendung einer Verbindung, erhältlich gemäss einem der Ansprüche 1 bis 14, oder eines pharmazeutisch verwendbaren Salzes davon zur Herstellung eines pharmazeutischen Präparats auf nichtchemischem Wege.